(19) Europäisches Patentamt
European Patent Office
Office européen des brevets

(11) Veröffentlichungsnummer: **0 120 392**
**A2**

(12) # EUROPÄISCHE PATENTANMELDUNG

(21) Anmeldenummer: **84102698.2**

(22) Anmeldetag: **13.03.84**

(51) Int. Cl.³: **C 07 D 407/06**
C 07 H 7/02, A 01 N 43/04
C 12 P 17/08, C 12 P 19/02
//(C07D407/06, 313/00, 309/00),
(C12P17/08, C12R1/04),
(C12P19/02, C12R1/04)

(30) Priorität: **23.03.83 DE 3310533**

(43) Veröffentlichungstag der Anmeldung:
**03.10.84 Patentblatt 84/40**

(84) Benannte Vertragsstaaten:
**BE CH DE FR GB IT LI NL**

(71) Anmelder: **BAYER AG**
**Konzernverwaltung RP Patentabteilung**
**D-5090 Leverkusen 1 Bayerwerk(DE)**

(72) Erfinder: **Hagenmaier, Hans-Paul, Prof. Dr.**
**Liegnitzer Strasse 8**
**D-7400 Tübingen(DE)**

(72) Erfinder: **Werner, Gerhard, Dr.**
**Dr. von Brentanostrasse 8**
**D-6941 Abtsteinach(DE)**

(72) Erfinder: **Drautz, Hannelore, Dr.**
**Tulpenweg 16**
**D-7406 Moessingen 2(DE)**

(72) Erfinder: **Holst, Hartwig, Dr.**
**Pestalozzistrasse 1**
**D-6301 Pohlheim 1(DE)**

(72) Erfinder: **Zähner, Hans, Prof. Dr.**
**Im Hopfengarten 13**
**D-7400 Tübingen(DE)**

(72) Erfinder: **Brandes, Wilhelm, Dr.**
**Eichendorffstrasse 3**
**D-5653 Leichlingen(DE)**

(72) Erfinder: **Reinecke, Paul, Dr.**
**Lessingstrasse 11**
**D-5090 Leverkusen 3(DE)**

(72) Erfinder: **Zoebelein, Gerhard, Dr.**
**Domblick 46**
**D-5090 Leverkusen 3(DE)**

(72) Erfinder: **Stendel, Wilhelm, Dr.**
**In den Birken 55**
**D-5600 Wuppertal 1(DE)**

(72) Erfinder: **Andrews, Peter, Dr.**
**Gellertweg 2**
**D-5600 Wuppertal 1(DE)**

(72) Erfinder: **Schaller, Klaus, Dr.**
**Am Sonnenschein 38**
**D-5600 Wuppertal 1(DE)**

(54) **Bafilomycine, und deren mikrobiologische Herstellung.**

(57) Die Erfindung betrifft neue Verbindungen der allgemeinen Formel I

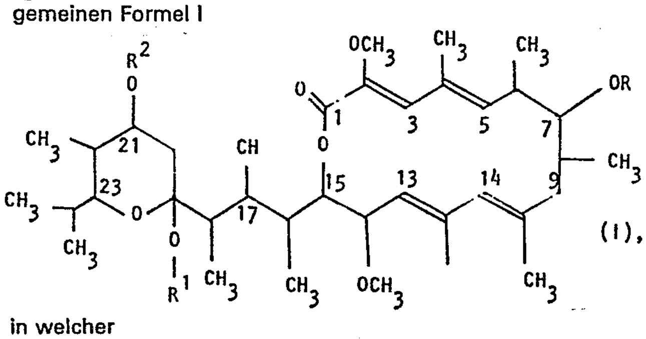

(I),

in welcher
R für Wasserstoff oder einen Acylrest steht,
$R^1$ für Wasserstoff oder $CH_3$ steht und
$R^2$ für Wasserstoff für einen Acylrest oder für die Gruppe

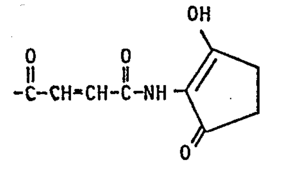

steht,

welche durch mikrobiologische Verfahren und gegebenenfalls anschließende Acylierung erhalten werden können, sowie deren Verwendung als Schädlings- und Parasitenbekämpfungsmittel.

./...

EP 0 120 392 A2

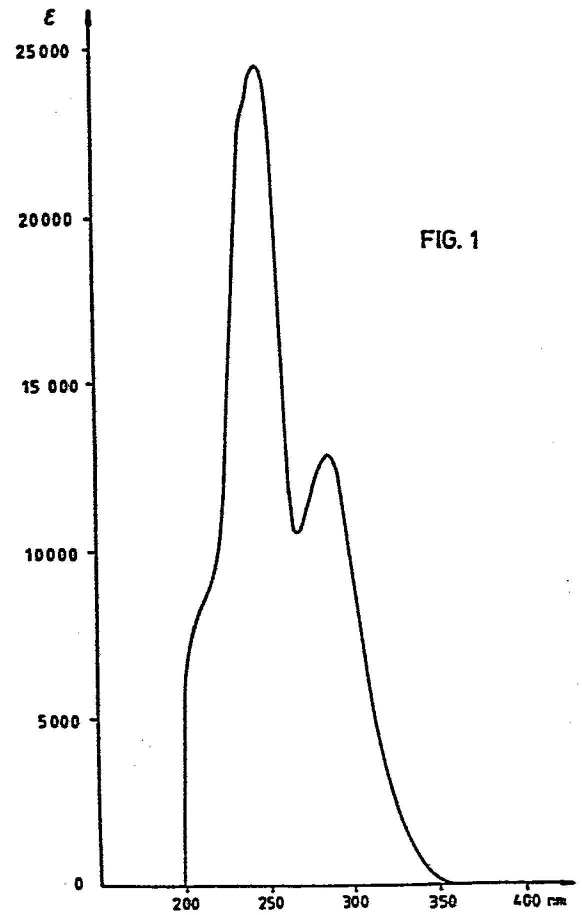


FIG. 1

BEZEICHNUNG GEÄNDERT
siehe Titelseite



BAYER AKTIENGESELLSCHAFT 5090 Leverkusen, Bayerwerk

S/Kü-c

Konzernverwaltung RP
Patentabteilung

# Organisch-chemische Verbindungen und Verfahren zu ihrer Herstellung

Die vorliegende Erfindung betrifft neue organischchemische Verbindungen, welche im folgenden kurz "Bafilomycine" genannt werden, Verfahren zu ihrer Hersellung auf im wesentlichen mikrobiologischem Wege und ihre Verwendung als Schädlings- und Parasitenbekämpfungsmittel.

Es wurden die neuen Bafilomycine, für die aufgrund der vorliegenden spektroskopischen und sonstigen analytischen Ergebnisse die folgende allgemeine Formel I

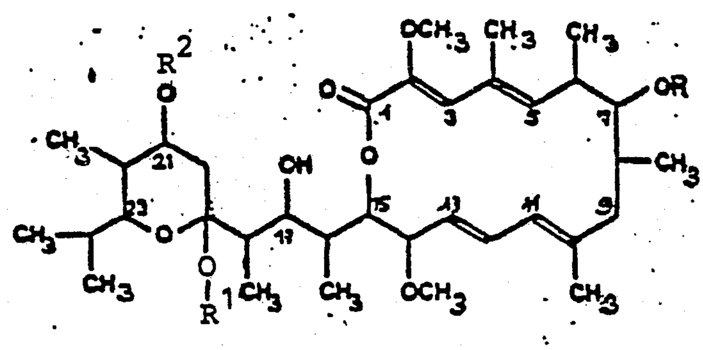

(I),

in welcher

R für Wasserstoff oder einen Acylrest steht,

$R^1$ für Wasserstoff oder $CH_3$ steht und

$R^2$ für Wasserstoff, für einen Acylrest oder für die

Gruppe 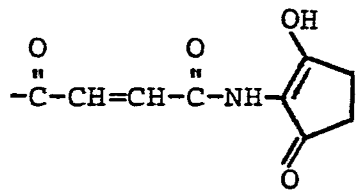 steht,

vorgeschlagen wird, gefunden.

Weiterhin wurde gefunden, daß die neuen Bafilomycine zur Bekämpfung von Schädlingen und Parasiten bei Pflanzen und Warmblütern verwendet werden können. Sie weisen eine hohe Wirksamkeit gegen Arthropoden (wie Insekten und Spinnentiere), Fungi und Würmer (Helminthes) auf. Die neuen Verbindungen und diese Verbindungen enthaltende Mittel können aufgrund dieser Eigenschaften besonders vorteilhaft im Pflanzenschutz, Vorratsschutz, im Hygienebereich, im Bereich der Parasitenbekämpfung beispielsweise in der Tierhaltung sowie im Materialschutz eingesetzt werden.

Die neuen Verbindungen der Formel I werden dadurch erhalten, daß man geeignete Mikroorganismen der Ordnung der Actinomycetales, vorzugsweise

der Familie Streptomycetaceae, insbesondere der Gattung Streptomyces in üblicher Weise in einem Nährmedium, welches assimilierbare Kohlenstoff- und Stickstoffquellen sowie Mineralsalze enthält, unter aeroben Bedingungen kultiviert, die gewünschten Verbindungen nach üblichen Methoden isoliert und gegebenenfalls acyliert.

Bei Kenntnis der Eigenschaften der neuen erfindungsgemäßen Verbindungen ist es mit Hilfe von üblichen chromatographischen, spektroskopischen und/oder mikrobiologischen (z.B. Hemmhoftest) Methoden leicht und rasch möglich in Routineverfahren solche geeigneten Mikroorganismenstämme auszusuchen, welche erfindungsgemäße Bafilomycine produzieren.

Für die mikrobiologische Herstellung der erfindungsgemäßen Verbindungen werden bevorzugt Streptomyces griseus Stämme eingesetzt. Ganz besonders bevorzugt werden hierbei die Streptomyces griseus Stämme Tü 1922, Tü 2437 und Tü 2599 sowie die Varianten und Mutanten dieser Stämme, welche die für die Ausführung der vorliegenden Erfindung wesentlichen Merkmale aufweisen.

Die Stämme Tü 1922, Tü 2437 und Tü 2599 wurden bei der Deutschen Sammlung von Mikroorganismen (DSM), Grisebachstraße 8, 3400 Göttingen, Bundesrepublik Deutschland hinterlegt:

| Stammkurzbezeichnung | Hinterlegung Aktenzeichen | Datum |
|---|---|---|
| Tü 1922 | DSM 2608 | 7.3.83 |
| Tü 2437 | DSM 2609 | 7.3.83 |
| Tü 2599 | DSM 2610 | 7.3.83 |

Beschreibung des Stammes Tü 1922:

Der Stamm Tü 1922 wurde aus einer Erdprobe aus Kaziranga (Assam), Indien isoliert und weist folgende Chrakteristika auf:

Sporenoberfläche: glatt
Sporengröße: 0,8 - 1,1 x 0,37 - 0,5 μ
Farbe des Luftmycels: grünlich-blau-grau (griseus)
Sporenketten: gerade leicht gewellt in Büscheln, keine Spiralen.

Melaninbildung: keine

Der Stamm gehört zweifelsfrei zur Art Streptomyces griseus.

0120392

Beschreibung der Stämme Tü 2437 und Tü 2599:

Der Stamm Tü 2437 stammt aus einer Erdprobe aus Thailand und der Stamm Tü 2599 wurde aus einer Erdprobe aus Peru isoliert.

Die Bestimmung erfolgte nach: R. Hütter: Systematik der Streptomyceten, Karger Verlag, Basel 1967

T.G. Pridham and
H. D. Tresner

Streptomycetaceae, in Bergey's Manual of Determinative Bacteriology, 8. Aufl. 1974, Williams & Wilkins Comp. Baltimore.

Beschreibung der Stämme:

Sporenoberfläche: glatt
Sporengrössen: 0,8 - 1,0 x 0,5 - 0,7 µ
Farbe des Luftmycel: anfänglich weiss, später grünlichgrau (griseus).

Sporenketten-Morphologie: Sporenketten gerade bis leicht gewellt, keine Spiralen, keine Quirle.

Melaninbildung: keine

Verwertung verschiedener C-Quellen:

| | |
|---|---|
| D-Glukose | + |
| 1-Arabinose | - |
| Sucrose | ± (-) |
| D-Xylose | + |
| m-Inosit | - |
| D-Mannit | + |
| D-Fructose | + |
| Rhamnose | - |
| Raffinose | ± |
| Cellulose | - |
| Galactose | + |

("+" bedeutet Verwertung und "-" bedeutet keine Verwertung).

Beide Stämme stimmen in allen Merkmalen überein und sind als typische Vertreter der Art Streptomyces griseus zu betrachten.

Die Verbindungen der Formel I, in welchen R und/oder $R^2$ für einen Acylrest stehen, können durch Acylierung der mikrobiologisch erzeugten Verbindungen erhalten werden, in welchen R und/oder $R^2$ Wasserstoff bedeuten.

Bevorzugt stehen die Acylreste R und $R^2$ für Alkyl-CO-Reste in welchen Alkyl geradkettig oder verzweigt ist und vorzugsweise 1 bis 6, insbesondere 1 bis 4 Kohlenstoffatome enthält, wobei beispielhaft Methyl, Ethyl, n- und i-Propyl und n-, sec-, i- und t-Butyl genannt seien. Ganz besonders bevorzugt stehen die Acylreste R und $R^2$ für die Acetylgruppe ($CH_3$-CO-).

Als bevorzugte Verbindungen der Formel I sollen die folgenden Bafilomycine genannt werden:

| Kurzbezeichnung: | Restbedeutung in Formel I: R | $R^1$ | $R^2$ |
|---|---|---|---|
| Bafilomycin $A_1$ | H | H | H |
| Bafilomycin $A_2$ | H | $CH_3$ | H |
| Bafilomycin $B_1$ | H | H | 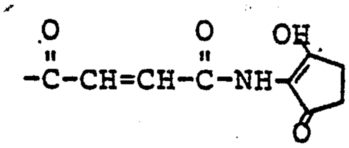 |
| Bafilomycin $B_2$ | H | $CH_3$ | " " " |
| Bafilomycin $D_1$ | H | H | $-COCH_3$ |
| Bafilomycin $D_1$) | $-COCH_3$ | H | $-COCH_3$ |
| Bafilomycin $D_2$ | H | $CH_3$ | $-COCH_3$ |
| Bafilomycin $D_2$) | $-COCH_3$ | $CH_3$ | $-COCH_3$ |

Unter den genannten Bafilomycinen sollen die Bafilomycine $A_1$, $A_2$, $B_1$ und $B_2$ als besonders bevorzugte erfindungsgemäße Verbindungen hervorgehoben werden. Ganz besonders bevorzugt werden Bafilomycin $A_1$ und $A_2$.

Wie bereits oben erläutert wurde, können die Bafilomycine $D_1$ und $D_1$) nach üblichen Acetylierungsmethoden (z.B. mit Acetylchlorid oder Acetanhydrid) aus Bafilomycin $A_1$ erhalten werden.

Die Strukturen der neuen erfindungsgemäßen Verbindungen wurden anhand von umfangreichen analytischen, insbesondere spektroskopischen Untersuchungen ermittelt. Da jedoch bei kompliziert gebauten Stoffen Irrtümer in der Interpretation der analytischen Befunde nicht immer völlig ausgeschlossen werden können, sollen die bevorzugten Bafilomycine auch durch einige physikalischchemische Daten charakterisiert werden:

1. Bafilomycin $A_1$:

| | |
|---|---|
| Aussehen: | farbloses, amorphes Pulver |
| Fp.: | 98 - 106°C (Zersetzung) |
| Löslichkeit: | leicht löslich in Methanol |
| | leicht löslich in Chloroform |
| | schwer löslich in Wasser |

Dünnschichtchromatogramm (Kieselgel):

$CHCl_3$ : $CH_3OH$ (9:1 Vol-Teile) Rf: 0,51

| | |
|---|---|
| Ethylmethylketon: | $CHCl_3$ (1:1 Vol-Teile) Rf: 0,42 |
| Ethylmethylketon: | Rf: 0,86 |
| Aceton: | Rf: 0,91 |

Molgewicht: 622

Summenformel (Elementaranalyse): $C_{35}H_{58}O_9$

| | | |
|---|---|---|
| Spektren: | UV | Fig. 1 |
| | IR | Fig. 2 |
| | NMR | Fig. 3 |

2. Bafilomycin $A_2$:

Aussehen: farbloses, amorphes Pulver
Fp.: 116 - 119°C (Zersetzung)
Löslichkeit: leicht löslich in Methanol
leicht löslich in Chloroform
schwer löslich in Wasser

Dünnschichtchromatogramm:
$CHCl_3$ : $CH_3OH$ (9:1 Vol-Teile) Rf: 0,52
Ethylmethylketon: $CHCl_3$ (1 : 1 Vol.-Teile) Rf: 0,42
Aceton: Rf: 0,91

Molgewicht: 636

Summenformel (Elementaranalyse): $C_{36}H_{60}O_9$

Spektren: UV Fig. 4
IR Fig. 5
NMR Fig. 6

3. Bafilomycin $B_1$:

Aussehen: intensiv gelb gefärbtes, amorphes Pulver
Fp.: 89 - 96°C (Zersetzung)
Löslichkeit: leicht löslich in Methanol
leicht löslich in Chloroform
leicht löslich in Pyridin
löslich in Essigsäureethylester
schwer löslich in Wasser

Dünnschichtchromatogramm (Kieselgel):

$CHCl_3$ : $CH_3OH$ (9:1 Vol.-Teile) Rf: 0,43
Ethylmethylketon: $CHCl_3$ (1:1 Vol.-Teile) Rf: 0,37
Ethylmethylketon: Rf: 0,78
Aceton: Rf: 0,76

Molgewicht: 815

Summenformel (Elementaranalyse); $C_{44}H_{65}O_{13}N$

Spektren: UV Fig. 7
IR Fig. 8
NMR Fig. 9

Die erfindungsgemäße Acylierung der Bafilomycine (R und $R^2$ = Wasserstoff) kann in üblicher Weise durchgeführt werden, wobei die üblichen Acylierungsmittel, wie Säurehalogenide oder Anhydride verwendet werden. Hierbei kann es zweckmäßig sein Basen (vorzugsweise organische Basen, wie Pyridin) zuzufügen. Vorzugsweise wird das Acylierungsmittel im Überschuß eingesetzt und die Reaktion unter schonenden Bedingungen, z.B. bei Raumtemperatur (ca. 17-25°C) vorgenommen. Das Acylierungsprodukt kann gewünschtenfalls durch chromatographische Methoden isoliert und gereinigt werden.

Die neuen Bafilomycine werden erfindungsgemäß durch die Fermentation geeigneter Mikroorganismenstämme, wie Tü 1922, Tü 2437 und Tü 2599 oder deren Mutanten oder Varianten erzeugt.

Das erfindungsgemäße Fermentationsverfahren kann mit Hilfe fester, halbfester oder flüssiger Nährmedien durchgeführt werden. Bevorzugt werden wässrig-flüssige Nährmedien verwendet.

Die Beimpfung der Nährmedien erfolgt nach allgemein üblichen Methoden, z. B. über Schrägröhrchen oder Kolbenkulturen.

Die Kultur erfolgt unter aeroben Bedingungen und kann gemäß den allgemein üblichen Methoden wie unter Verwendung von Schüttelkulturen z.B. in Schüttelkolben, von luftbewegten Kulturen oder von Submerskulturen durchgeführt werden. Bevorzugt erfolgt die Kultivierung im aeroben Submersverfahren in belüfteten Fermentern, z.B. in üblichen Submersfermentationstanks. Es ist möglich, die Kultur kontinuierlich oder diskontinuierlich durchzuführen. Vorzugsweise wird diskontinuierlich gearbeitet.

Die Kultur kann in allen Nährmedien durchgeführt werden, welche bekannterweise zur Kultivierung von Mikroorganismen der Ordnung Actinomycetales verwendet werden. Das Nährmedium muß eine oder mehrere assimilierbare Kohlenstoffquellen und Stickstoffquellen sowie Mineralsalze enthalten, wobei diese Produkte in Form von definierten Einzelbestandteilen, aber auch in Form von komplexen Gemischen, wie sie insbesondere biologische Produkte verschiedenen Ursprungs darstellen, vorliegen können. Als

Kohlenstoffquellen kommen alle üblichen Kohlenstoffquellen infrage. Beispielsweise seien Kohlehydrate, insbesondere Polysaccharide, wie Stärke oder Dextrine, Disaccharide, wie Maltose oder Rohrzucker, Monosaccharide, wie Glucose oder Xylose, Zuckeralkohole, wie Mannit oder Glycerin sowie natürlich vorkommende Gemische, wie Malzextrakt, Melasse oder Molkepulver genannt. Als Stickstoffquellen kommen alle üblichen organischen und anorganischen Stickstoffquellen infrage. Beispielsweise seien Eiweißstoffe, Eiweißhydrolysate, Aminosäuren, wie Glutaminsäure, Asparaginsäure, Arginin, Lysin, Ornithin oder Serin, Nucleosidbasen, wie Cytosin oder Uracil sowie Sojabohnenmehl, Baumwollsamenmehl, Linsenmehl, Erbsenmehl, lösliche und unlösliche pflanzliche Proteine, Maisquellwasser, Hefeextrakt, Peptone und Fleischextrakt sowie Ammoniumsalze und Nitrate, z.B. $NH_4Cl$, $(NH_4)_2SO_4$, Na $NO_3$ und $KNO_3$ aufgeführt. Die Mineralsalze, welche im Nährmedium enthalten sein sollten, liefern z.B. folgende Ionen:

$Mg^{++}$, $Na^{+}$, $K^{+}$, $Ca^{++}$, $NH_4^{+}$, $Cl^{-}$, $SO_4^{--}$, $PO_4^{---}$ und $NO_3^{-}$

sowie Ionen der üblichen Spurenelemente, wie Cu, Fe, Mn, Mo, Zn, Co, Ni. Falls die Kohlenstoff- oder Stickstoffquellen bzw. das verwendete Wasser nicht ausreichend diese Salze bzw. Spurenelemente enthalten, ist es zweckmäßig, das Nährmedium entsprechend zu ergänzen. Die Zusammensetzung der Nährmedien kann in weiten Bereichen variiert werden. Art und Zusammensetzung der Nährmedien

werden im allgemeinen davon abhängig sein, welche Bestandteile jeweils besonders günstig zur Verfügung stehen. Im allgemeinen enthalten die Nährlösungen vorzugsweise etwa 0,5 bis 8 %, insbesondere 0,6 bis 6 % Kohlenstoffquellen, vorzugsweise etwa 0,5 bis 4 %, insbesondere 0,5 bis 2 % Stickstoffquellen und vorzugsweise etwa 0,001 bis 0,5 %, insbesondere 0,003 bis 0,3 % Mineralsalze.

Bei der Durchführung des Verfahrens kann es günstig sein, zu Beginn der Kultivierung nur relativ geringe Konzentrationen der löslichen Nährlösungsbestandteile zu verwenden und dann im Laufe der ersten 3 Kultivierungstage durch häufigere Zusätze diese Bestandteile in Form steriler, relativ konzentrierter Lösung dem Kulturansatz fraktioniert zuzufüttern.

Der pH-Wert der wachsenden Kulturen sollte vorzugsweise zwischen etwa 5 und etwa 10, insbesondere zwischen 6,5 und 9,5 gehalten werden. Ein zu starker pH-Abfall in den sauren Bereich kann durch Zusätze einer organischen oder anorganischen Base, vorzugsweise von $CaCO_3$ vermieden werden. Wie in der Fermentationstechnologie üblich, kann auch eine automatische pH-Regulierung durchgeführt werden, bei der sterile organische oder anorganische Säure, z.B. $H_2SO_4$, oder sterile Lauge, z.B. NaOH in Abständen in die Kulturlösung eingespritzt wird.

Es ist zweckmäßig sicherzustellen, daß die Mikroorganismen ausreichend mit Sauerstoff sowie den Nährstoffen in

Kontakt gebracht werden. Dies kann nach den allgemein üblichen Methoden wie Schütteln und Rühren erfolgen.

Die Züchtungstemperatur kann zwischen etwa 15 und etwa 40°C, vorzugsweise zwischen 20 und 35°C liegen, besonders bevorzugt liegt sie bei etwa 28°C. Die Dauer der Züchtung kann stark variiert werden, wobei z.B. die Zusammensetzung des Nährmediums und die Züchtungstemperatur eine Rolle spielen. Die jeweiligen optimalen Bedingungen können von jedem Fachmann auf dem mikrobiologischen Gebiet leicht festgelegt werden.

Es hat sich herausgestellt, daß die Menge der sich in der Kulturbrühe anreichernden erfindungsgemäßen Verbindung im allgemeinen ihr Maximum etwa 1 bis 10, vorzugsweise etwa 3 bis 5 Tage nach Züchtungsbeginn erreicht. Das gewünschte Endprodukt der Fermentation kann mit Hilfe von dünnschichtchromatographischen und hochdruckflüssigkeitschromatographischen Untersuchungen oder im Plattendiffusionstest mit einem geeigneten Pilz als Teststamm bestimmt werden.

Wie allgemein bei mikrobiologischen Verfahren sollten Fremdinfektionen der Kulturmedien vermieden werden. Hierzu werden die üblichen Vorkehrungen getroffen, wie Sterilisation der Nährmedien, der Kulturgefäße sowie der für die Belüftung notwendigen Luft. Zur Sterilisation der Vorrichtungen können z.B. die Dampf- als auch die Trockensterilisation verwendet werden, wobei die

Temperaturen vorzugsweise bei 100 bis 140°C, insbesondere bei 120 bis 130°C liegen können.

Falls bei der Kultivierung in unerwünschter Menge Schaum entsteht, können die üblichen chemischen Schaumdämpfungsmittel, z.B. flüssige Fette und Öle, Öl-Wasser-Emulsionen, Paraffine, höhere Alkohole, wie Octadecanol, Siliconöle, Polyoxyethylen- bzw. Polyoxypropylenverbindungen (z.B. in Mengen bis etwa 1 %) zugesetzt werden. Schaum kann auch mit Hilfe der üblichen mechanischen Vorrichtungen (welche z.B. Zentrifugalkräfte benutzen) gedämpft oder beseitigt werden.

Die erfindungsgemäßen Verbindungen können aus dem Kulturmedium nach allgemein üblichen physikalisch-chemischen Methoden isoliert werden. Die Isolierung kann z.B. gemäß den üblichen Extraktionsverfahren, Fällungsverfahren und/oder Chromatographieverfahren erfolgen. Die isolierten Stoffe können mit Hilfe der genannten Methoden auch feingereinigt werden. Für viele Fälle ist jedoch eine Feinreinigung nicht erforderlich, da gegebenenfalls vorhandene geringfügige Verunreinigungen die Wirksamkeit der Verbindungen nicht nachteilig beeinflussen. Bei allen Isolierungs- und Reinigungsoperationen ist darauf zu achten, daß pH-Werte nicht über eine längere Zeit im sauren Bereich liegen. Vorzugsweise werden pH-Werte zwischen 7 und 10 eingehalten. Zur Erniedrigung des pH-Wertes können anorganische und organische Basen verwendet werden, wie Alkalibasen z.B. NaOH, KOH, oder organische Amine, wie Triethylamin.

Um bei den o.a. Isolierungs- und Reinigungsmethoden die Fraktionen herauszufinden, in welchen die erfindungsgemäßen Verbindungen in höchster Konzentration bzw. Reinheit vorliegen, können die üblichen physikalisch-chemischen Methoden, z.B. Messen einer charakteristischen Bande im Spektrum oder der $R_f$-Werte, Bestimmung der antimikrobiellen Aktivität u.s.w. herangezogen werden. Diese Methoden können auch verwendet werden, um in Routineverfahren geeignete Mikroorganismen aufzufinden.

Die Isolierung und Reinigung der erfindungsgemäßen Verbindungen kann z.B. im Falle, daß ein flüssiges wäßriges Nährmedium verwendet wird, wie folgt vorgenommen werden: Nach seiner Anreicherung im Kulturüberstand werden Kulturfiltrat und Mycel mit üblichen Methoden separiert (z.B. Zentrifugation).

Aus dem Kulturfiltrat können die erfindungsgemäßen Verbindungen mit Hilfe von üblichen Extraktionsverfahren, Fällungsverfahren und/oder Chromatographieverfahren isoliert und gegebenenfalls gereinigt werden. Die Chromatographie kann in Form der Säulenchromatographie durchgeführt werden. Mit gutem Erfolg läßt sich auch die Hochdruckflüssigkeitschromatographie (HPLC) einsetzen. Als Adsorptionsmittel können die üblichen anorganischen oder organischen Adsorptionsmittel eingesetzt werden, wie z.B. Aluminiumoxid, Kieselgel, Magnesiumsilikat, Aktivkohle, Cellulose, Cellulosederivate, Kunstharze wie Polyamide, z.B. acetyliertes Polyamid

oder Dextrangele. Als Laufmittel können die verschiedensten Lösungsmittel oder Lösungsmittelgemische verwendet werden, in welchen die erfindungsgemäßen Verbindungen löslich sind. Bevorzugt werden Essigsäureethylester, Chloroform und Methanol oder ihre Gemische (beispielsweise Gemische aus Chloroform und Methanol oder aus Essigsäureethylester und Chloroform) eingesetzt. Die Bafilomycine sollten nur solange wie erforderlich mit Methanol in Kontakt gebracht werden.

Bevorzugt werden zur Isolierung der erfindungsgemäßen Verbindungen Chromatographie-Verfahren verwendet, z.B. unspezifische Adsorption an Sorbentien wie Kieselgel oder andererseits Geldiffusionschromatographie. Dies sind die von der Reinigung schlecht wasserlöslicher Naturstoffe her bekannte Methoden.

Aus ihren Lösungen können die erfindungsgemäßen Verbindungen nach den üblichen Methoden, z.B. Verdampfen des Lösungsmittels, Gefriertrocknung usw. erhalten werden.

In einer bevorzugten Ausführungsform der Erfindung wird das bei der aeroben Kultur der Stämme bei etwa 27°C erhaltene Fermentationsgut (Kulturbrühe und Mycel) mit einem polaren Lösungsmittel, wie Essigsäureethylester mehrfach, vorzugsweise 3 mal extrahiert. Die vereinigten Extrakte werden mit Natriumsulfat getrocknet und das Lösungsmittel (vorzugsweise im Hochvakuum bei etwa 0°C) abdestilliert.

Das Bafilomycin-Rohprodukt wird anschließend einer präparativen fraktionierten Säulenchromatographie unterworfen. Als Adsorptionsmittel wird hierbei Kieselgel bevorzugt. Bevorzugte Eluenten sind Mischungen polarer Lösungsmittel, insbesondere von Chloroform und Methanol, wobei ein Verhältnis von etwa 1:1 (Vol.-Teile) besonders günstige Ergebnisse liefert. Die Fraktionen können leicht anhand dünnschichtchromatographischer Untersuchungen festgelegt werden. Auf diese Weise werden die Roh-Bafilomycine $A_1/A_2$ und $B_1/B_2$ erhalten. Die Roh-Bafilomycine werden weiter säulenchromatographisch (vorzugsweise auf Kieselgel) mit den bevorzugten Eluenten Chloroform/Methanol und im Falle von $B_1/B_2$ zusätzlich von Chloroform/Essigsäureethylester weitergereinigt. Eine Feinreinigung der auf diese Weise erhaltenen (bereits recht reinen) Produkte erfolgt zweckmäßigerweise durch eine Hochdruckflüssigkeitschromatographie (HPLC) mit üblichen Geräten, wobei als Füllmaterial vorzugsweise übliche "reversed phase" - Materialien, wie mit Paraffin modifiziertes Kieselgel und als Eluente vorzugsweise Methanol/Wasser-Mischungen dienen.

Die Wirkstoffe eignen sich bei guter Pflanzenverträglichkeit und günstiger Warmblütertoxizität zur Bekämpfung vc tierischen Schädlingen, insbesondere Insekten, Spinnentieren und Nematoden, die in der Landwirtschaft, in Forsten, im Vorrats- und Materialschutz sowie auf dem Hygienesektor vorkommen. Sie sind gegen normal sensible ur

resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.
Aus der Ordnung der Diplopoda z.B. Blaniulus guttulatus.
Aus der Ordnung der Chilopoda z.B. Geophilus carpophagus, Scutigera spec.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.
Aus der Ordnung der Dermaptera z.B. Forficula auricularia.
Aus der Ordnung der Isoptera z.B. Reticulitermes spp..
Aus der Ordnung der Anoplura z.B. Phylloxera vastatrix, Pemphigus spp., Pediculus humanus corporis, Haematopinus spp., Linognathus spp.
Aus der Ordnung der Mallophaga z.B. Trichodectes spp., Damalinea spp.
Aus der Ordnung der Thysanoptera z.B. Hercinothrips femoralis, Thrips tabaci.
Aus der Ordnung der Heteroptera z.B. Eurygaster spp.,

Dysdercus intermedius, Piesma quadrata, Cimex lectularius, Rhodnius prolixus, Triatoma spp.
Aus der Ordnung der Homoptera z.B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.
Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.
Aus der Ordnung der Coleoptera z.B. Anobium punctatum, Rhizopertha dominica, Bruchidius obtectus, Acanthoscelides obtectus, Hylotrupes bajulus, Agelastica alni,

Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp..

Aus der Ordnung der Arachnida z.B. Scorpio maurus, Latrodectus mactans.

Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Zu den pflanzenparasitären Nematoden gehören Pratylenchus spp., Radopholus similis, Ditylenchus dipsaci, Tylenchulus semipenetrans, Heterodera spp., Meloidogyne spp., Aphelenchoides spp., Longidorus spp., Xiphinema spp., Trichodorus spp..

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Bakterizide Mittel werden im Pflanzenschutz zur Bekämpfung von Pseudomonadaceae, Rhizobiaceae, Enterobacteriaceae, Corynebacteriaceae und Streptomycetaceae eingesetzt.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Die erfindungsgemäßen Wirkstoffe eignen sich auch zur Bekämpfung von Ekto- und Endoparasiten bei Warmblütern, beispielsweise auf dem Gebiet der Tierhaltung und Viehzucht, wobei durch die Bekämpfung der Schädlinge bessere Ergebnisse, z.B. höhere Milchleistungen, höheres Gewicht, längere Lebensdauer usw. erreicht werden können.

Die Anwendung der erfindungsgemäßen Wirkstoffe geschieht auf diesen Gebieten in bekannter Weise, wie durch orale Anwendung in Form von beispielsweise Tabletten, Kapseln, Tränken, Granulaten, durch dermale Anwendung in Form beispielsweise des Tauchens (Dippen), Sprühens (Sprayen), Aufgießens (pour-on and spot-on) und des Einpuderns sowie durch parenterale Anwendung in Form beispielsweise der Injektion.

Es werden auf diesen Gebieten z.B. auch Zecken und solche Arthropoden erfaßt, bei welchen Teile ihrer Entwicklung im Tier ablaufen. Darüberhinaus sind die erfindungsgemäßen Verbindungen auch zur Bekämpfung von Würmern - insbesondere solche der Klasse Nematoda - geeignet, die Parasiten von Warmblütern sind. Außerdem können die erfindungsgemäßen Verbindungen zur Bekämpfung von dermalen Pilzinfektionen bei Warmblütern verwendet werden.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo- und Metallphthalocyaninfarb-

stoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Die erfindungsgemäßen Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Die neuen Bafilomycine können nicht nur direkt als Wirkstoffe verwendet werden, sondern stellen auch wertvolle Ausgangssubstanzen für die Synthese weiterer neuer Wirkstoffe dar (insbesondere die Bafilomycine $A_1$ und $A_2$ sind hierfür geeignet).

Die Herstellung der erfindungsgemäßen Verbindungen soll anhand der folgenden Beispiele erläutert werden. Wo nicht anders angegeben, beziehen sich alle %-Angaben auf Gewichtsprozente. Essigester bedeutet Essigsäureethylester.

I. Beispiele für die Fermentation der Stämme Tü 1922, Tü 2437 und Tü 2599

1. Fermentation des Stammes Tü 1922

Der Stamm Tü 1922 wird bei etwa 4°C im Kühlraum auf einem Schrägagarröhrchen gehalten. Der hierzu verwendete HA-Agar enthält je 1 4 g Hefeextrakt, 10 g Malz, 4 g Dextrose und 15 g Agar (Rest: Wasser) und hat einen pH-Wert von 7,3. Sporenlösungen des Stammes Tü 1922 werden bei -20°C aufbewahrt.

Für die Fermentation wird eine Nährlösung (NL 111) eingesetzt, welche, außer Leitungswasser, 2 % Fleischmehl, 2 % Malzextrakt und 1 % Kalk enthält und einen pH-Wert von 7,2 aufweist.

a) Durchführung im Kolbenmaßstab

500 ml Erlenmeyerkolben mit einem seitlichen Einstich, die 100 ml Nährlösung (NL 111) enthalten, werden aus einer Schrägagarkultur beimpft und auf der rotierenden Schüttelmaschine bei 120 rpm (Umdrehungen pro Minute) und 27°C für 4 Tage inkubiert. Als Vorkultur für den 10 l-Fermenter dient eine 48 h alte Kultur.

b) Durchführung im 10 l-Fermenter

Die Fermentation wird in einem 10 l-Fermenter (Modell MF-14 New Brunswick, Scientific Co., New Brunswick, USA) durchgeführt, der 9 l Nährlösung (NL 111) enthält. Die Sterilisation erfolgt für 30 min bei 134°C. Bei 27°C Inkubationstemperatur und 250 rpm des Rührers werden 4 l Luft (als Vorfermenter für den 100 l-Fermenter werden 2 l Luft pro Minute verwendet) pro Minute in die Kulturflüssigkeit eingeleitet. Durch wiederholte Zugabe weniger Tropfen ethanolischer Polyollösung wird die Schaumentwicklung unterdrückt. Die Impfmaterialmenge beträgt 5 % eines der unter a) beschriebenen Kulturen.
Die Ernte erfolgt nach ca. 70 Stunden.

c) Durchführung im 100 l-Fermenter

Als Impfmaterial wird der Inhalt eines 10 l-Fermenters (vgl. b) nach 48-stündiger Fermentation verwendet. Der 100 l-Fermenter enthält insgesamt 90 l Material (einschließlich Impfmaterial). Als Nährlösung wird das Nährmedium NL 111 (vgl. oben) eingesetzt. Die Inkubationszeit bis zur maximalen Bafilomycinproduktion beträgt ca. 70 Stunden bei 200 rpm des Plattrührers wobei 50 l Luft pro Minute eingeleitet werden und die Temperatur auf etwa 27°C gehalten wird.

2. Fermentation des Stammes Tü 2437

Die Aufbewahrung des Stammes Tü 2437 erfolgt, wie oben beim Stamm Tü 1922 beschrieben.

Als Nährlösung für die Fermentation wird eine Zubereitung verwendet, welche aus Leitungswasser, 2 % Sojamehl und 2 % Laktose besteht und welche einen pH-Wert von 7,5 aufweist.

a) Durchführung im Kolbenmaßstab

Die Durchführung erfolgt, wie beim Stamm Tü 1922 beschrieben, wobei jedoch die Sojamehl/Laktose-Nährlösung verwendet wird.

b) Durchführung im 100 l-Fermenter

Die Durchführung erfolgt wie beim Stamm Tü 1922 beschrieben. Es wird jedoch die Sojamehl/Laktose-Nährlösung eingesetzt. Unter Rühren mit einem Blattrührer (240 rpm) werden 360 l Luft je Stunde eingeleitet und die Fermentation bei 27°C für 24,5 Stunden durchgeführt.

c) Durchführung in 100 l-Fermenter

Der nach b) erhaltene Ansatz wird als Impfmaterial in den 100 l-Fermenter gebracht. Mit der obigen Sojamehl/Laktose-Nährlösung wird

0120392

auf 90 l aufgefüllt. In die Mischung werden unter Rühren mit einem Blattrührer (200 rpm) 3400 l Luft pro Stunde eingeleitet und die Fermentation unter Zusatz von ca. 30 ml ethanolischen Polyollösung (als Entschäumer) für 71,5 Stunden bei 27°C vorgenommen.

3. Fermentation des Stammes Tü 2599

Die Aufbewahrung des Stammes Tü 2599 erfolgt, wie beim Stamm Tü 1922 oben beschrieben.

Die Durchführung der Fermentation im Kolbenmaßstab sowie in den 10- und 100 l-Fermentern erfolgt genau, wie bei der Fermentation des Stammes Tü 2437 beschrieben.

II. Beispiele für die Aufarbeitung der gemäß I erhaltenen Kulturbrühen

1. Herstellung der Bafilomycin-Rohextraktlösung

Das Fermentationsgut wird durch die Zugabe von Essigsäure unter Rühren auf einen pH-Wert von 6 (Stämme Tü 2437 und Tü 2599) oder durch die Zugabe von 10 %-wäßriger NaOH auf einen pH-Wert von 10 (Stamm Tü 1922) eingestellt. Anschließend wird das gebildete Mycel mit Hilfe einer Filterpresse abgetrennt. Das Mycel kann

direkt verworfen werden oder aber vorzugsweise zwecks einer Bafilomycin-Ausbeuteerhöhung zuvor extrahiert werden. Hierzu wird als Extraktionsmittel eine Mischung aus Methanol und Aceton (1:1 Vol.-Teile) verwendet. Man setzt auf 1 kg Mycel insgesamt etwa 2000 ml des Lösungsmittelgemisches ein. Das Mycel wird mit dem Lösungsmittelgemisch 3 mal extrahiert und anschließend verworfen. Von der Lösung wird das Lösungsmittelgemisch im Hochvakuum bei 0°C abdestilliert. Der Rückstand wird mit Essigester (etwa 50 ml Essigester je 1 g Rückstand) aufgenommen und zur Abtrennung fester Bestandteile filtriert.

Die vom Mycel befreite Kulturbrühe wird 3 mal mit insgesamt etwa 20 Vol.-Teilen Essigester je 100 Vol.-Teilen Kulturbrühe extrahiert. Die Essigesterextrakte aus Kulturbrühe und Mycel werden vereinigt und mit Natriumsulfat getrocknet. Die Lösung wird durch Destillation im Hochvakuum bei 0°C auf etwa 1/10 des ursprünglichen Volumens eingeengt. Im Falle der Fermentierung im 100 l-Fermenter erhält man somit etwa 2 Liter Essigesterlösung (Rohextraktlösung).

2. Grobreinigung des Bafilomycin-Rohextraktes

2 l der nach 1 erhaltenen Rohextraktlösung werden mit wenig (500 ml) Wasser pH 7,0 ausgeschüttelt. Der Wasserextrakt wird 2 mal mit Essigester rückextrahiert. Alle Essigesterphasen werden vereinigt, mit Natriumsulfat getrocknet, abfiltriert und das Lösungsmittel im Hochvakuum bei 0°C bis auf 500 ml abdestilliert. Aus diesem konzentrierten Extrakt fällt nach 24 h bei 0°C ein gelblicher Niederschlag aus. Dieser wird abgesaugt und mit eiskaltem Essigester gewaschen. Der Niederschlag wird verworfen.

Das Filtrat wird 2 mal mit je 100 ml Wasser (pH 7,0) gewaschen, die Wasserphasen 1 mal mit Essigester rückextrahiert, alle Essigesterphasen vereinigt, mit Natriumsulfat getrocknet, abfiltriert und im Hochvakuum bei 0°C vollständig vom Lösungsmittel befreit.

Hatte der Essigesterextrakt noch eine hellgelbe Farbe, zeigt der Rohextrakt eine braune Farbe. Im Fall der Fermentation von Tü 1922 im 100 l-Fermenter werden auf diese Weise etwa 4,7 g Rohextrakt erhalten.

4,7 g dieses Rohproduktes werden in 30 ml Chloroform/Methanol 1:1 (Vol.-Teile) gelöst und über eine 400 g Kieselgelsäule (Kieselgel 60; Korngröße 0,063 - 0,200 mm; MERCK) mit dem Gemisch Chloroform/Methanol 9:1 (Vol.-Teile) als Eluent in Fraktionen zu je 15 ml aufgetrennt. Ab Fraktion 60 wird auf 100 % Methanol gewechselt. Säule: 200 cm x 35 mm.

Aufgrund der DC-Kontrolle werden folgende Fraktionen (Fr.) vereinigt und das Lösungsmittel abdestilliert:

| | | |
|---|---|---|
| Fr. 1 - 6 | 0,70 g | Fette |
| Fr. 7 - 11 | 2,60 g | $A_1/A_2$ und Fette, wenig $B_1/B_2$ |
| Fr. 12 - 19 | 0,50 g | $B_1/B_2$ und wenig $A_1/A_2$ |
| Fr. 20 - 28 | 0,15 g | $B_1/B_2$ |
| Fr. 29 - 43 | 0,40 g | keine Bafilomycin-Komponenten |
| Fr. 44 - 60 | 0,14 g | keine Bafilomycin-Komponenten |
| Fr. 61 - 91 | 0,27 g | unerwünschte Produkte |

Material aus den Fraktionen 7 - 11 (2,6 g) wird als $A_1/A_2$ roh und aus den Fraktionen 12 - 28 (0,65 g) als $B_1/B_2$ roh bezeichnet.

Die Fraktionen 44 - 91 werden verworfen.

Die dünnschichtchromatographische Kontrolle (DC-Kontrolle) erfolgt auf MERCK DC-Fertigplatten Kieselgel 60 F 254, Schichtdicke 0,25 mm, im Laufmittelsystem $CHCl_3/CH_3OH$ 9:1 (Vol.-Teile).

Die aktiven Komponenten können durch UV (254/366 nm), Besprühen mit

a) conz. $HCl/CH_3OH$ (30/70 Vol.-Teile) (Rotfärbung) und
b) Jodsprühreagenz (0,2 g Jod + 0,4 KJ in 50 ml Wasser und 50 ml Ethanol) charakterisiert werden.

| Hauptkomponenten | Rf-Wert |
|---|---|
| $B_1/B_2$ | 0,38 - 0,43 |
| $A_1/A_2$ | 0,54 |

3. Feinreinigung der Bafilomycine

a) Bafilomycine $A_1$ und $A_2$

$A_1/A_2$ roh wird in 20 ml Chloroform gelöst und erneut über eine 300 g Kieselgelsäule (150 cm x 3 cm, Kieselgel 60, 0,063 - 0,200 mm, WOELM) mit Chloroform/Methanol 95:5 (Vol.-Teile) aufgetrennt. Die Fraktionen 15 - 29 enthalten 0,68 g vorgereinigtes $A_1/A_2$, die Fraktionen 21 - 29 0,06 g $B_1/B_2$ Komponenten. Die Hauptmenge sind nicht näher charakterisierte Fette in den Fraktionen 1 - 14 (1,4 g).

Bei der Vorreinigung von $B_1/B_2$ fallen noch zusätzlich 0,08 g $A_1/A_2$ Komponenten an. Nach den HPLC-Untersuchungen enthalten die vereinigten Fraktionen 14 - 17 zu 65 % $A_1$, während die 0,08 g zu 85 % $A_2$ enthalten (Fraktionsvolumen: 15 ml).

Die beiden vorgereinigten Verbindungen $A_1$ und $A_2$ werden vereinigt (0,76 g) und über eine 150 g Kieselgelsäule (150 cm x 2 cm, Kieselgel 60, 0,063 - 0,200 mm, WOELM) mit dem Eluent Ethylmethylketon/Chloroform 1:1 (Vol.-Teile) aufgetrennt. Die Fraktionen (10 ml) 1 - 6 enthalten in 0,58 g neben Fetten $A_1$, die Fraktionen 7 - 12 (0,15 g) $A_2$. Zur endgül-

tigen Abtrennung der Fette werden die vereinigten Fraktionen 1 - 6 über präparative HPLC aufgetrennt.

Mit der Fraktion 7 - 12 wird ebenso verfahren. Die Schnittfraktionen beider präparativer Trennungen wurden vereinigt und erneut über eine präparative RP-18 Säule aufgetrennt. Man eluiert 45 mg $A_1$ (92 %ig) und 36 mg $A_2$ (95 %ig). Die Stoffe wurden nach dem Abdestillieren des Lösungsmittels erhalten.

Einige ergänzende Angaben über die HPLC-Trennung (High Pressure Liquid Chromatography), welche mit handelsüblichen Geräten und Materialien durchgeführt werden kann:

| | |
|---|---|
| Pumpe: | KNAUER Typ 52.00 |
| Injektor: | RHEODYN 7125 mit einer 2 ml Probenschleife |
| Säule: | KNAUER 25 cm x 16 mm im Durchmesser |
| Füllmaterial: | MERCK RP-18 10 µm (Kieselgel, modifiziert mit Aliphat ($C_{18}$)) |
| Eluent: | Methanol/Wasser 80:20 (Vol.-Teile) pH 7,0 |
| Fluß: | 10 ml/min |
| Detektion: | UV-Absorption bei 290 nm |
| Fraktionsvolumen: | 15 ml |

In den Fraktionen 5 - 7 sind 35 mg $A_1$ enthalten, in den Fraktionen 8 - 10 15 mg Mischung $A_2$ und Verunreinigungen. Die Auf der Säule retardierten Fette werden mit Methanol über Nacht eluiert und verworfen.

b) Verbindungen $B_1$ und $B_2$

Zu den 0,65 g $B_1/B_2$ (vgl. Grobreinigung) kommen noch 0,06 g der gleichen Mischung von der Feinreinigung von $A_1/A_2$. Diese vereinigten Fraktionen (0,71 g) werden über eine 150 g Kieselgelsäule (Kieselgel 60, 0,063 - 0,200 mm, MERCK) mit dem Eluent Chloroform/Methanol 9:1 (Vol.-Teile) aufgetrennt (Säulendimension: 150 cm x 2 cm , Fraktionsvolumen: 10 ml). Die Fraktionen 16 - 28 enthalten 0,22 g $B_1/B_2$, aus den Fraktionen 8 - 13 können 0,08 g $A_1/A_2$ isoliert werden.

Die vorgereinigten 0,22 g $B_1/B_2$-Komponenten werden über eine 50 g Kieselgelsäule (100 x 1 cm, Kieselgel 60, 0,063 - 0,200 mm, WOELM), erst mit dem Eluentgemisch Chloroform/Essigester 1:1 (Vol.-Teile) und nach einem Säulenvolumen mit 100 % Essigester aufgetrennt. Fraktionsvolumen: 10 ml. Die Fraktionen 18 - 25 enthalten 0,09 g $B_1$ (94 %ig) und die Fraktionen 26 - 38 enthalten 0,04 g $B_2$ (67 %ig), welche durch das Abdestillieren des Lösungsmittels erhalten werden.

Bafilomycin $B_1$ wurde oben beschrieben. Das Bafilomycin $B_2$ unterschied sich in Dünnschichtchromatogramm vom Bafilomycin $B_1$:

| Laufmittel | Rf | |
|---|---|---|
| | $B_1$ | $B_2$ |
| $CHCl_3$-$CH_3OH$ 9 : 1 (Vol.-Teile) | 0,43 | 0,38 |
| EMK - $CHCl_3$ 1 : 1 (Vol.-Teile) | 0,37 | 0,40 |
| EMK | 0,78 | 0,81 |
| Aceton | 0,76 | 0,82 |

EMK = Ethylmethylketon

0120392

III. Beispiel für die Acylierung der Bafilomycine

Zu einer Lösung von 23 mg $A_1$ (87 %ig, enthält 12 % $A_2$) in 3 ml Pyridin werden 1 ml Acetanhydrid hinzugefügt und 24 h bei 20°C im Dunkeln gerührt. Anschließend gießt man auf 60 ml Eis und extrahiert 3 mal mit je 10 ml Chloroform. Die organischen Phasen werden mit Wasser gewaschen, mit Natriumsulfat getrocknet und im Vakuum vom Lösungsmittel befreit. Das gelbliche Reaktionsgemisch wird sofort in 1 ml Methanol gelöst und auf einer HPLC-Säule (25 cm x 16 mm i.D.) an RP-18 10 µm MERCK mit Methanol/Wasser 80:20 (Vol.-Teile) pH 7,0 getrennt, Fluß 10 ml/min., Detektion; UV-Absorption bei 290 nm.

Auf diese Weise werden folgende Verbindungen der Formel I erhalten, deren Struktur durch spektroskopische Methoden (IR-, NMR-, Massenspektren) ermittelt wurde:

a) R = H; $R^1$ = H; $R^2$ = $-COCH_3$ Ausbeute: 6 mg
b) R = $COCH_3$; $R^1$ = H; $R^2$ = $-COCH_3$ Ausbeute: 10 mg
c) R = $COCH_3$; $R^1$ = $CH_3$; $R^2$ = $-COCH_3$ Ausbeute: 2 mg

<u>Erläuterung der Figuren 1 bis 9:</u>

1. Allgemeine Erläuterungen

| | | |
|---|---|---|
| 1. | UV-Spektren: | Fig 1: $A_1$; Fig 4: $A_2$; Fig. 7: $B_1$ |
| | Abzisse: | Wellenlänge (nm) |
| | Ordinate: | Extinktion (molarer Extinktionskoeffizient $\varepsilon$) |
| | Lösungsmittel: | Methanol |
| 2. | IR-Spektren: | (in KBr): Fig. 2: $A_1$; Fig. 5: $A_2$; Fig. 8: $B_1$ |
| | Abzisse: | Wellenlänge (µm) bzw. Wellenzahl ($cm^{-1}$) |
| | Ordinate: | Durchlässigkeit (%) |
| 3. | NMR-Spektren: | $^1H$-NMR-Spektren, aufgenommen bei 400 MHz<br>Interner Standard: Tetramethylsilan (TMS)<br>Chemical shift in ppm<br>Lösungsmittel und Konzentrationen:<br>Fig. 3 ($A_1$): $CDCl_3$, c = 10 mg/ml<br>Fig. 6 ($A_2$): $CDCl_3$, c = 12 mg/ml<br>Fig. 9 ($B_2$): $CDCl_3$, c = 10 mg/ml |

Erläuterung einiger aufgeführter Firmennamen und Warenzeichen

| | |
|---|---|
| MERCK | bedeutet Fa. E. Merck, Darmstadt, Bundesrepublik Deutschland |
| WOELM | bedeutet Fa. Woelm, Eschwege, Bundesrepublik Deutschland |
| KNAUER | bedeutet Fa. Knauer, Berlin Bundesrepublik Deutschland |
| RHEODYN | ist ein Warenzeichen der Fa. Knauer, Berlin, Bundesrepublik Deutschland |

Die biologische Wirksamkeit der erfindungsgemäßen Verbindungen soll anhand der folgenden Beispiele erläutert werden:

Beispiel A

Phaedon-Test

Lösungsmittel: 3 Gewichtsteile Aceton
Emulgator: 1 Gewichtsteil Alkylarylpolyglykolether

Zur Herstellung einer zweckmäßigen Wirkstoffzubereitung vermischt man 1 Gew.-Teil Wirkstoff mit der angegebenen Menge Lösungsmittel und der angegebenen Menge Emulgator und verdünnt das Konzentrat mit Wasser auf die gewünschten Konzentrationen.

Kohlpflanzen (Brassica oleracea) werden durch Sprühen mit der Wirkstoffzubereitung der gewünschten Konzentration behandelt. Nach Antrocknen der Spritzbrühe wird von der behandelten Pflanze ein Blatt entnommen, in eine Plastikdose gelegt und mit Larven des Meerrettichkäfers (Phaedon cochleariae) besetzt. Nach 2 bis 4 Tagen wird jeweils ein weiteres Blatt von derselben Pflanze für die Nachfütterung verwendet.

Nach der gewünschten Zeit wird die Abtötung in % bestimmt. Dabei bedeutet 100 %, daß alle Tiere abgetötet wurden; 0 % bedeutet, daß keine Tiere abgetötet wurden.

Bei diesem Test zeigten nach 14 Tagen z. B. bei einer Konzentration von 0,004 % das Bafilomycin $B_1$ eine Abtötung von 100 % und das Bafilomycin $A_1$ bei einer Konzentration von 0,02 % eine Abtötung von 100 %.

Beispiel B

Plutella-Test

Lösungsmittel: 3 Gewichtsteile Aceton
Emulgator: 1 Gewichtsteil Alkylarylpolyglykolether

Zur Herstellung einer zweckmäßigen Wirkstoffzubereitung vermischt man 1 Gew.-Teil Wirkstoff mit der angegebenen Menge Lösungsmittel und der angegebenen Menge Emulgator und verdünnt das Konzentrat mit Wasser auf die gewünschten Konzentrationen.

Kohlpflanzen (Brassica oleracea) werden durch Sprühen mit der Wirkstoffzubereitung der gewünschten Konzentration behandelt. Nach Antrocknen der Spritzbrühe wird von der behandelten Pflanze ein Blatt entnommen, in eine Plastikdose gelegt und mit Raupen der Kohlschabe (Plutella maculipennis) besetzt. Nach 2 bis 4 Tagen wird jeweils ein weiteres Blatt von derselben Pflanze für die Nachfütterung verwendet.

Nach der gewünschten Zeit wird die Abtötung in % bestimmt. Dabei bedeutet 100 %, daß alle Tiere abgetötet wurden; 0 % bedeutet, daß keine Tiere abgetötet wurden.

Bei diesem Test zeigten nach 14 Tagen z. B. bei einer Konzentration von 0,004 % das Bafilomycin $B_1$ eine Abtötung von 100 % und bei einer Konzentration von 0,05 % das Bafilomycin $A_1$ eine Abtötung von 100 %.

Beispiel C

Entwicklungshemmtest mit Dysdercus intermedius (Baumwollkapselwanze)

Lösungsmittel: 3 Gewichtsteile Aceton
Emulgator: 1 Gewichtsteil Alkylarylpolyglykolether

Zur Herstellung einer zweckmäßigen Wirkstoffzubereitung vermischt man 1 Gew.-Teil Wirkstoff mit der angegebenen Menge Lösungsmittel und der angegebenen Menge Emulgator und verdünnt das Konzentrat mit Wasser auf die gewünschten Konzentrationen.

In Plastikdosen werden je 10 Nymphen der Baumwollkapselwanze einige Baumwollsamen und ein mit der Wirkstoffzubereitung getränktes Watteröllchen angeboten. Nach der gewünschten Zeit wird die Abtötung in % bestimmt. Dabei bedeutet 100 %, daß alle Tiere abgetötet wurden; 0 % bedeutet, daß keine Tiere abgetötet wurden.

Bei diesem Test zeigten nach 14 Tagen z. B. bei einer Konzentration von 0,004 % das Bafilomycin $B_1$ eine Abtötung von 100 % und bei einer Konzentration von 0,01 % das Bafilomycin $A_1$ eine Abtötung von 100 %.

0120392

Beispiel D

Entwicklungshemmtest mit Ceratitis capitata (Mittelmeerfruchtfliege)

Lösungsmittel: 3 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschten Konzentrationen.

Je 20 Eier der Mittelmeerfruchtfliege werden auf Kunstfutterbrei in ein Döschen gelegt. Das Futter ist mit Wirkstoff in der angegebenen Konzentration behandelt. Die Summe der abgetöteten Eier, Larven, Puppen und Imagines bezogen auf die Zahl eingesetzter Eier ergibt die Abtötung in %.

Dabei bedeutet 100 %, daß alle Tiere abgetötet wurden; 0 % bedeutet, daß keine Tiere abgetötet wurden.

Bei diesem Test zeigten nach 21 Tagen z. B. bei einer Konzentration von 0,00016 % das Bafilomycin $B_1$ eine Abtötung von 100 % und bei einer Konzentration von 0,0008 % das Bafilomycin $A_1$ eine Abtötung von 100 %.

0120392

Beispiel E

Test mit Psoroptes cuniculi

Lösungsmittel: 35 Gewichtsteile Ethylenglykolmonomethylether
35 Gewichtsteile Nonylphenolpolyglykolether

Zur Herstellung einer zweckmäßigen Wirkstoffzubereitung vermischt man 3 Gew.-Teile Wirkstoff mit 7 Gew.-Teilen des oben angegebenen Lösungsmittel-Gemisches und verdünnt das so erhaltene Konzentrat mit Wasser auf die gewünschte Konzentration.

Etwa 10-25 Psoroptes cuniculi werden in 1 ml der zu testenden Wirkstoffzubereitung gebracht, die in Tablettennester einer Tiefziehverpackung pipettiert wurden. Nach 24 Stunden wird der Abtötungsgrad bestimmt.

Bei diesem Test zeigten z.B. bei einer Konzentration von 80 ppm die Bafilomycine $A_1$ und $B_1$ eine Abtötung von 100 %.

Beispiel F

Test mit Lucilia cuprina res.-Larven

Emulgator: 35 Gewichtsteile Ethylenglykolmonomethylether
35 Gewichtsteile Nonylphenolpolyglykolether

Zur Herstellung einer zweckmäßigen Wirkstoffzubereitung vermischt man 3 Gew.-Teile Wirkstoff mit 7 Gew.-Teilen des oben angegebenen Lösungsmittel-Gemisches und verdünnt das so erhaltene Konzentrat mit Wasser auf die jeweils gewünschte Konzentration.

Etwa 20 Lucilia cuprina res.-Larven werden in ein Teströhrchen gebracht, welches ca. 1 $cm^2$ Pferdefleisch und 0,5 ml der Wirkstoffzubereitung enthält. Nach 24 Stunden wird der Abtötungsgrad bestimmt.

Bei diesem Test zeigte z. B. bei einer Konzentration von 80 ppm das Bafilomycin $B_1$ eine abtötende Wirkung von etwa 95 %.

Beispiel G

Tetranychus-Test (resistent)

Lösungsmittel: 3 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Bohnenpflanzen (Phaseolus vulgaris), die stark von allen Entwicklungsstadien der gemeinen Spinnmilbe oder Bohnenspinnmilbe (Tetranychus urticae) befallen sind, werden durch Tauchen in die Wirkstoffzubereitung der gewünschten Konzentration behandelt.

Nach der gewünschten Zeit wird die Abtötung in % bestimmt. Dabei bedeutet 100 %, daß alle Spinnmilben abgetötet wurden; 0 % bedeutet, daß keine Spinnmilben abgetötet wurden.

Bei diesem Test zeigt z. B. Bafilomycin $A_1$ bei einer Konzentration von 0,1 % nach 7 Tagen eine Abtötung von 95 % und Bafilomycin $B_1$ bei 0,02 % nach 7 Tagen eine Abtötung von 100 %.

Beispiel H

Cochliobolus sativus-Test (Gerste) / protektiv

Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gew.-Teil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Konidiensuspension von Cochliobolus sativus besprüht. Die Pflanzen verbleiben 48 Stunden bei 20°C und 100 % rel. Luftfeuchtigkeit in einer Inkubationskabine.

Die Pflanzen werden in einem Gewächshaus bei einer Temperatur von ca. 20°C und einer relativen Luftfeuchtigkeit von ca. 80 % aufgestellt.

7 Tage nach der Inokulation erfolgt die Auswertung.

Im Vergleich zur unbehandelten Kontrolle (100 % Befall) ergaben die Behandlung mit Bafilomycin $B_1$ nur einen Befall von 12,5 % (Wirkstoffkonzentrationen: 0,025 %) und die Behandlung mit Bafilomycin $A_1$ nur einen Befall von 41,2 % (Wirkstoffkonzentration: 0,025 %).

Beispiel I

Pyrenophora teres-Test (Gerste) / protektiv

Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gew.-Teil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Konidiensuspension von Pyrenophora teres besprüht. Die Pflanzen verbleiben 48 Stunden bei 20°C und 100 % rel. Luftfeuchtigkeit in einer Inkubationskabine.

Die Pflanzen werden in einem Gewächshaus bei einer Temperatur von ca. 20°C und einer relativen Luftfeuchtigkeit von ca. 80 % aufgestellt.

7 Tage nach der Inokulation erfolgt die Auswertung.

Im Vergleich zur unbehandelten Kontrolle (100 % Befall) ergaben die Behandlungen mit Bafilomycin $A_1$ und mit Bafilomycin $B_1$ nur einen Befall von 12,5 % (Wirkstoffkonzentrationen 0,025 %).

Beispiel K

Botrytis-Test (Bohne) / protektiv

Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden auf jedes Blatt 2 kleine mit Botrytis cinerea bewachsene Agarstückchen aufgelegt. Die inokulierten Pflanzen werden in einer abgedunkelten, feuchten Kammer bei 20°C aufgestellt. 3 Tage nach der Inokulation wird die Größe der Befallsflecken auf den Blättern ausgewertet.

Im Vergleich zur unbehandelten Kontrolle (100 % Befall) ergab Behandlung mit Bafilomycin $B_1$ nur einen Befall mit 4 % bei Wirkstoffkonzentrationen von 250 ppm.

Beispiel L

Phytophthora-Test (Tomate) /protektiv

Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Sporensuspension von Phytophthora infestans inokuliert.

Die Pflanzen werden in einer Inkubationskabine mit 100 % relativer Luftfeuchtigkeit und ca. 20 °C aufgestellt.

3 Tage nach der Inokulation erfolgt die Auswertung.

Im Vergleich zur unbehandelten Kontrolle (90 % Befall) ergab bei einer Wirkstoffkonzentration von 250 ppm die Behandlung mit Bafilomycin $B_1$ nur einen Befall von 9 %.

Beispiel M

Pellicularia-Test (Reis)

Lösungsmittel: 12,5 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf Wirksamkeit werden junge Reispflanzen im 3- bis 4-Blattstadium tropfnaß gespritzt. Die Pflanzen verbleiben bis zum Abtrocknen im Gewächshaus. Anschließend werden die Pflanzen mit Pellicularia sasakii inokuliert und bei 25°C und 100 % relativer Luftfeuchtigkeit aufgestellt.

5 bis 8 Tage nach der Inokulation erfolgt die Auswertung des Krankheitsbefalles.

Im Vergleich zur unbehandelten Kontrolle (100 % Befall) ergab eine Behandlung mit Bafilomycin $B_1$ einen Befall von 0 % (Wirkstoffkonzentration 0,025 %).

Das erfindungsgemäße Bafilomycin $B_1$ zeigte außerdem in Tests mit Puccinia recondita an Getreide, Pyricularia oryzae an Reis, Venturia inaequalis am Apfel gute Wirksamkeiten.

Beispiel N

Antimykotische in-vitro-Wirksamkeit

Versuchsbeschreibung:

Die in-vitro-Prüfungen wurden im Reihenverdünnungstest mit Keiminokula von durchschnittlich 5 x $10^3$ bis $10^4$ Keimen/ml Substrat durchgeführt. Als Nährmedium dienten

a) für Dermatophyten und Schimmelpilze: Sabouroud's milieu d'épreuve

b) für Hefen: Fleischextrakt-Traubenzucker-Bouillon.

Die Bebrütungstemperatur betrug 28 bis 37°C, die Bebrütungsdauer lag bei 24 bis 96 Stunden bei Hefen und 96 Stunden bei Dermatophyten und Schimmelpilzen.

In diesem Test zeigte die erfindungsgemäße Verbindung $B_1$ eine gute Wirkung.

Beispiel O

Nematoden in vitro Test

Geprüft wird die Abtötung und Hemmung der Vermehrung des Nematoden Caenorhabditis elegans in flüssigem Medium in Gegenwart von Bakterien, die den Nematoden als Nahrung dienen.

In diesem Test hemmten die Bafilomycine $A_1$ und $B_1$ die Vermehrung von C. elegans in einer Konzentration von 25 µg/ml um mehr als 90 %.

Beispiel P

Puccinia-Test (Weizen) / protektiv

Lösungsmittel: 100 Gewichtsteile Dimethylformamid
Emulgator: 0,25 Gewichtsteil Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit einer Sporensuspension von Puccinia recondita in einer 0,1 %igen wäßrigen Agarlösung inokuliert. Nach Antrocknen besprüht man die Pflanzen mit der Wirkstoffzubereitung taufeucht. Die Pflanzen verbleiben 24 Stunden bei 20°C und 100 % rel. Luftfeuchtigkeit in einer Inkubationskabine.

Die Pflanzen werden in einem Gewächshaus bei einer Temperatur von ca. 20°C und einer relativen Luftfeuchtigkeit von ca. 80 % aufgestellt, um die Entwicklung von Rostpusteln zu begünstigen. 10 Tage nach der Inokulation erfolgt die Auswertung.

Im Vergleich zur unbehandelten Kontrolle (100 % Befall) zeigten z. B. bei einer Konzentration von 0,025 % die behandelten Pflanzen bei Bafilomycin $A_1$ 0 % Befall und bei Bafilomycin $B_1$ 12,5 % Befall.

Patentansprüche

1. Verbindungen der allgemeinen Formel I

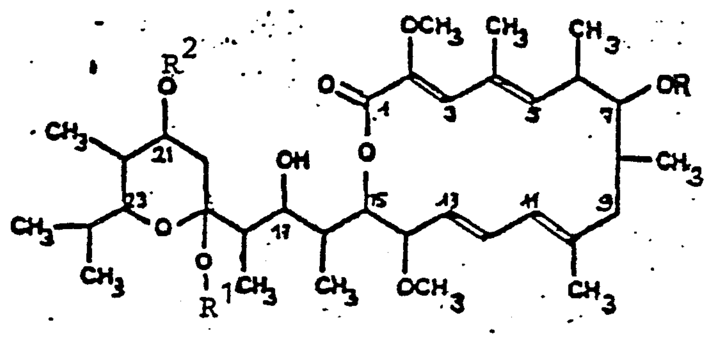

(I)

in welcher

R für Wasserstoff oder einen Acylrest steht,

$R^1$ für Wasserstoff oder $CH_3$ steht und

$R^2$ für Wasserstoff, für einen Acylrest oder für

die Gruppe 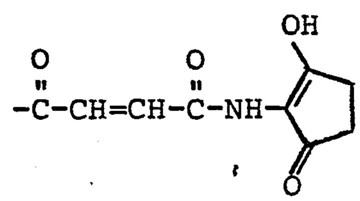

steht.

2. Verbindungen der allgemeinen Formel I, gemäß Anspruch 1, in welcher

   a) R, $R^1$ und $R^2$ für Wasserstoff stehen;

   b) R und $R^2$ für Wasserstoff und $R^1$ für $CH_3$ stehen;

   c) R und $R^1$ für Wasserstoffe und $R^2$ für den Rest 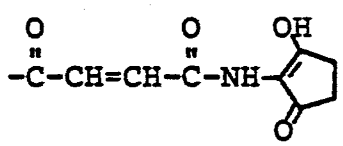 stehen;

   d) R für Wasserstoff, $R^1$ für $CH_3$ und $R^2$ für den Rest 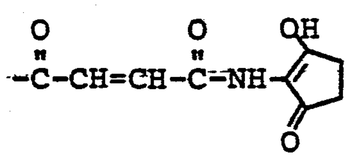 stehen;

   e) R und $R^1$ für Wasserstoff und $R^2$ für $-COCH_3$ stehen;

   f) R für $-COCH_3$, $R^1$ für Wasserstoff und $R^2$ für $-COCH_3$ stehen.

3. Verbindungen der allgemeinen Formel I, gemäß Anspruch 1, in welcher

a) R, $R^1$ und $R^2$ für Wasserstoff stehen und

b) R und $R^1$ für Wasserstoff und $R^2$ für den Rest

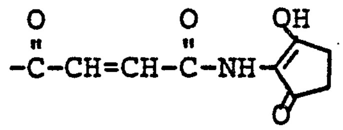 stehen.

4. Bafilomycin $A_1$ mit folgenden Eigenschaften:

| | |
|---|---|
| Aussehen: | farbloses, amorphes Pulver |
| Fp.: | 98 - 106°C (Zersetzung) |
| Löslichkeit: | leicht löslich in Methanol |
| | leicht löslich in Chloroform |
| | schwer löslich in Wasser |

Dünnschichtchromatogramm (Kieselgel):

$CHCl_3$ : $CH_3OH$ (9 : 1 Vol.-Teile) Rf: 0,51

| | |
|---|---|
| Ethylmethylketon: | $CHCl_3$ (1:1 Vol.-Teile) Rf: 0,42 |
| Ethylmethylketon: | Rf: 0,86 |
| Aceton: | Rf: 0,91 |

Molgewicht: 622

Summenformel (Elementaranalyse): $C_{35}H_{58}O_9$

Spektren: UV Fig. 1
IR Fig. 2
NMR Fig. 3

5. Bafilomycin $A_2$:

Aussehen: farbloses, amorphes Pulver
Fp.: 116 - 119°C (Zersetzung)
Löslichkeit: leicht löslich in Methanol
leicht löslich in Chloroform
schwer löslich in Wasser

Dünnschichtchromatogramm:
$CHCl_3$ : $CH_3OH$ (9:1 Vol-Teile) Rf: 0,52
Ethylmethylketon: $CHCl_3$ (1:1 Vol-Teile) Rf: 0,42
Aceton: Rf: 0,91

Molgewicht: 636

Summenformel (Elementaranalyse): $C_{36}H_{60}O_9$

Spektren: UV Fig. 4
IR Fig. 5
NMR Fig. 6

6. Bafilomycin $B_1$ mit folgenden Eigenschaften:

Aussehen: intensiv gelb gefärbtes, amorphes Pulver

Fp.: 89 - 96°C (Zersetzung)
Löslichkeit: leicht löslich in Methanol
leicht löslich in Chloroform
leicht löslich in Pyridin
löslich in Essigsäureethylester
schwer löslich in Wasser

Dünnschichtchromatogramm (Kieselgel):
$CHCl_3$ : $CH_3OH$ (9:1 Vol.-Teile) Rf: 0,43
Ethylmethylketon: $CHCl_3$ (1:1 Vol.-Teile) Rf: 0,37
Ethylmethylketon: Rf: 0,78
Aceton: Rf: 0,76

Molgewicht: 815

Summenformel (Elementaranalyse); $C_{44}H_{65}O_{13}N$

Spektren: UV Fig. 7
IR Fig. 8
NMR Fig. 9

7. Verbindungen gemäß den Ansprüchen 1 bis 6 (ausgenommen die Verbindungen der allgemeinen Formel I, in welchen R und/oder $R^2$ für einen Acylrest steht), erhältlich durch die Fermentation der Stämme Tü 1922, Tü 2437 und Tü 2599 oder der Mutanten und Varianten dieser Stämme.

8. Verfahren zur Herstellung der Verbindungen der allgemeinen Formel (I)

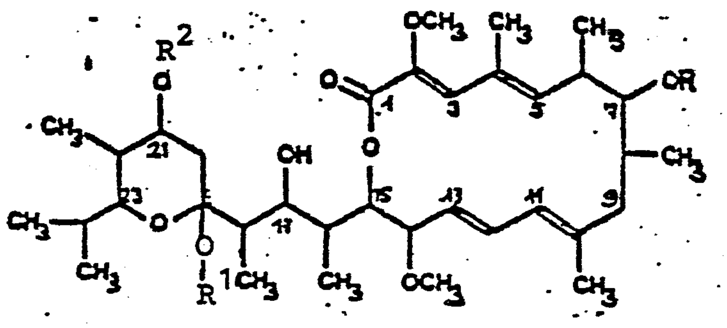

(I)

in welcher

R für Wasserstoff oder einen Acylrest steht,

$R^1$ für Wasserstoff oder $CH_3$ steht und

$R^2$ für Wasserstoff, für einen Acylrest oder für

die Gruppe 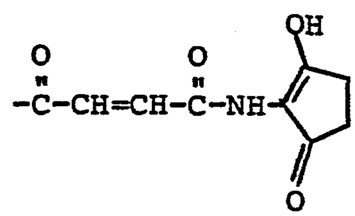

steht,

dadurch gekennzeichnet, daß man geeignete Mikroorganismen der Ordnung der Actinomycetales, vorzugsweise der Familie Streptomycetaceae, insbesondere der Gattung Streptomyces in üblicher Weise in einem Nährmedium, welches assimilierbare Kohlenstoff- und Stickstoffquellen sowie Mineralsalze enthält, unter aeroben Bedingungen kultiviert, die gewünschten Verbindungen nach üblichen Methoden isoliert und gegebenenfalls acyliert.

9. Verfahren zur Herstellung der Verbindungen gemäß den Ansprüchen 4 bis 6, dadurch gekennzeichnet, daß man geeignete Mikroorganismen der Ordnung der Actinomycetales, vorzugsweise der Familie Streptomycetaceae, insbesondere der Gattung Streptomyces und ganz besonders bevorzugt Stämme der Art Streptomyces griseus in üblicher Weise in einem Nährmedium, welches assimilierbare Kohlenstoff- und Stickstoffquellen sowie Mineralsalze enthält, unter aeroben Bedingungen kultiviert und die gewünschten Verbindungen nach üblichen Methoden isoliert.

10. Verfahren gemäß den Ansprüchen 8 und 9, dadurch gekennzeichnet, daß man für die Kultivierung die Stämme Tü 1922, Tü 2437 und Tü 2599 oder die Mutanten und Varianten dieser Stämme einsetzt.

11. Schädlingsbekämpfungsmittel, gekennzeichnet durch einen Gehalt an mindestens einer Verbindung der Formel I gemäß den Ansprüchen 1 und 8 oder einer Verbindung gemäß den Ansprüchen 4 bis 7.

12. Verwendung von Verbindungen der Formel I gemäß den Ansprüchen 1 und 8 oder von Verbindungen gemäß den Ansprüchen 4 bis 7 zur Bekämpfung von Schädlingen und Parasiten, insbesondere Arthropoden (wie Insekten und Spinnentieren), Pilzen oder Würmern.

13. Verfahren zur Bekämpfung von Schädlingen und Parasiten, dadurch gekennzeichnet, daß man Verbindungen der Formel I gemäß den Ansprüchen 1 oder 8 oder Verbindungen gemäß den Ansprüchen 4 bis 7 auf die Schädlinge, vorzugsweise Arthropoden (wie Insekten oder Spinnentiere), Pilze oder Würmer oder ihren Lebensraum einwirken läßt.

14. Verfahren zur Herstellung von Schädlings- und Parasitenbekämpfungsmitteln, dadurch gekennzeichnet, daß man Verbindungen der Formel I gemäß den Ansprüchen 1 oder 8 oder Verbindungen gemäß den Ansprüchen 5 und 6 mit Streckmitteln und/oder oberflächenaktiven Mitteln vermischt.

15. Mikroorganismen Tü 1922, Tü 2437 und Tü 2599 und deren Mutanten und Varianten.

16. Verbindungen der allgemeinen Formel I gemäß Anspruch 1 zur Bekämpfung von Erkrankungen.

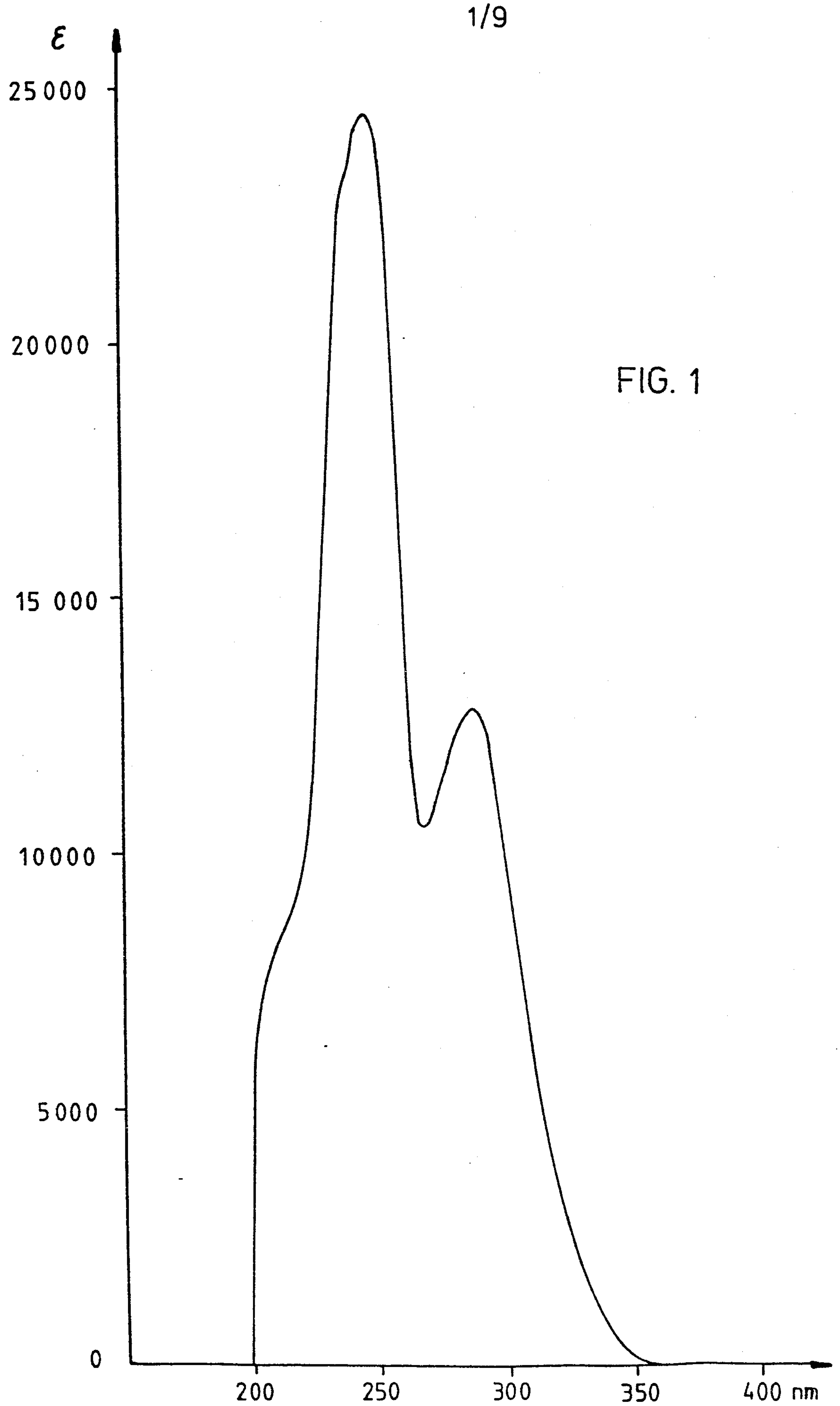


FIG. 1

0120392

2/9

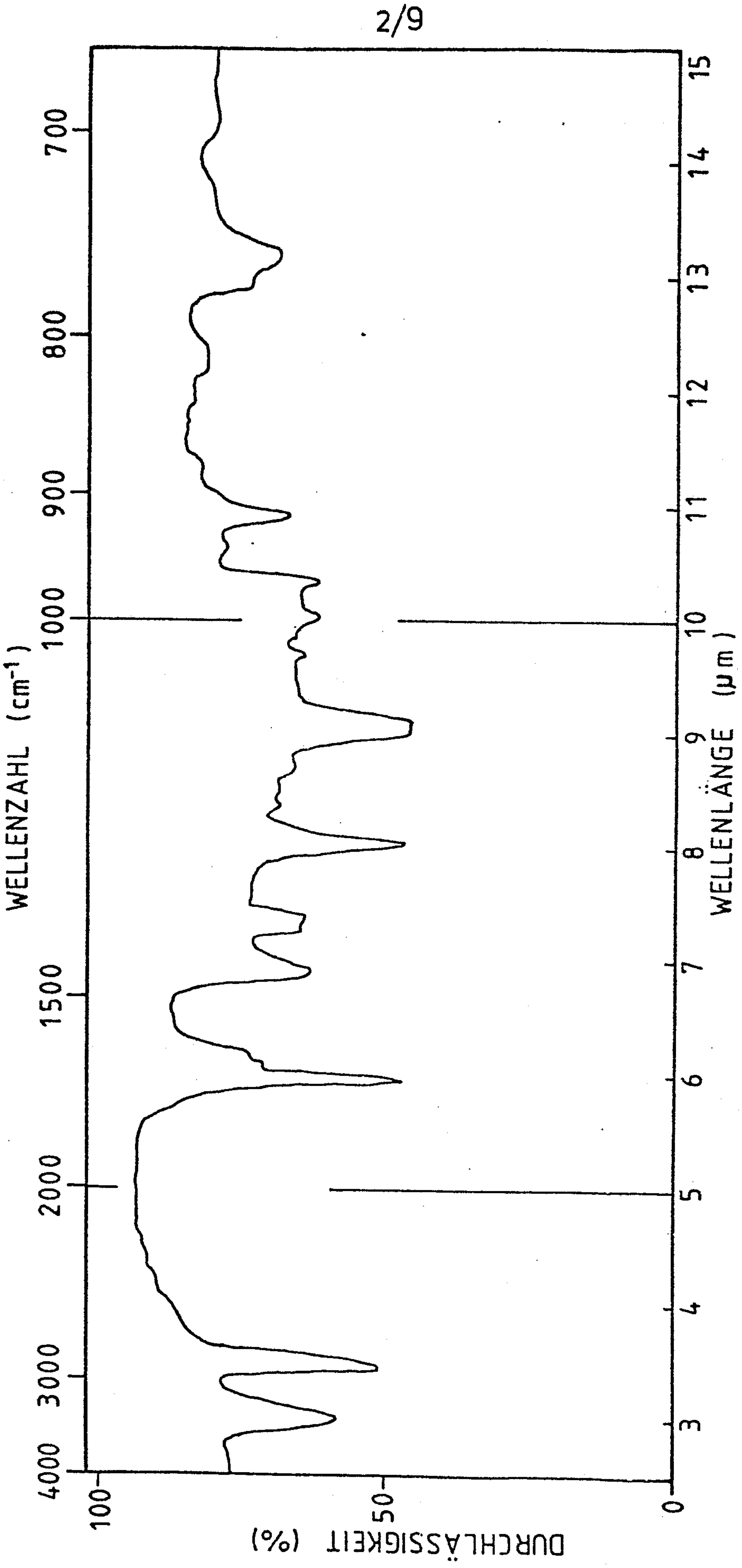


FIG. 2

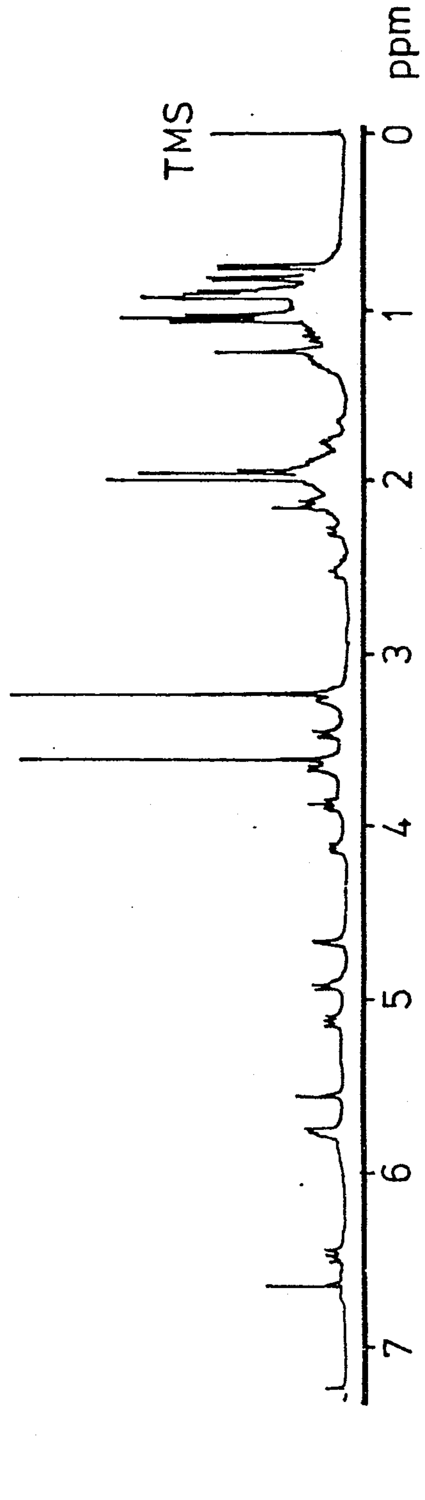


FIG. 3

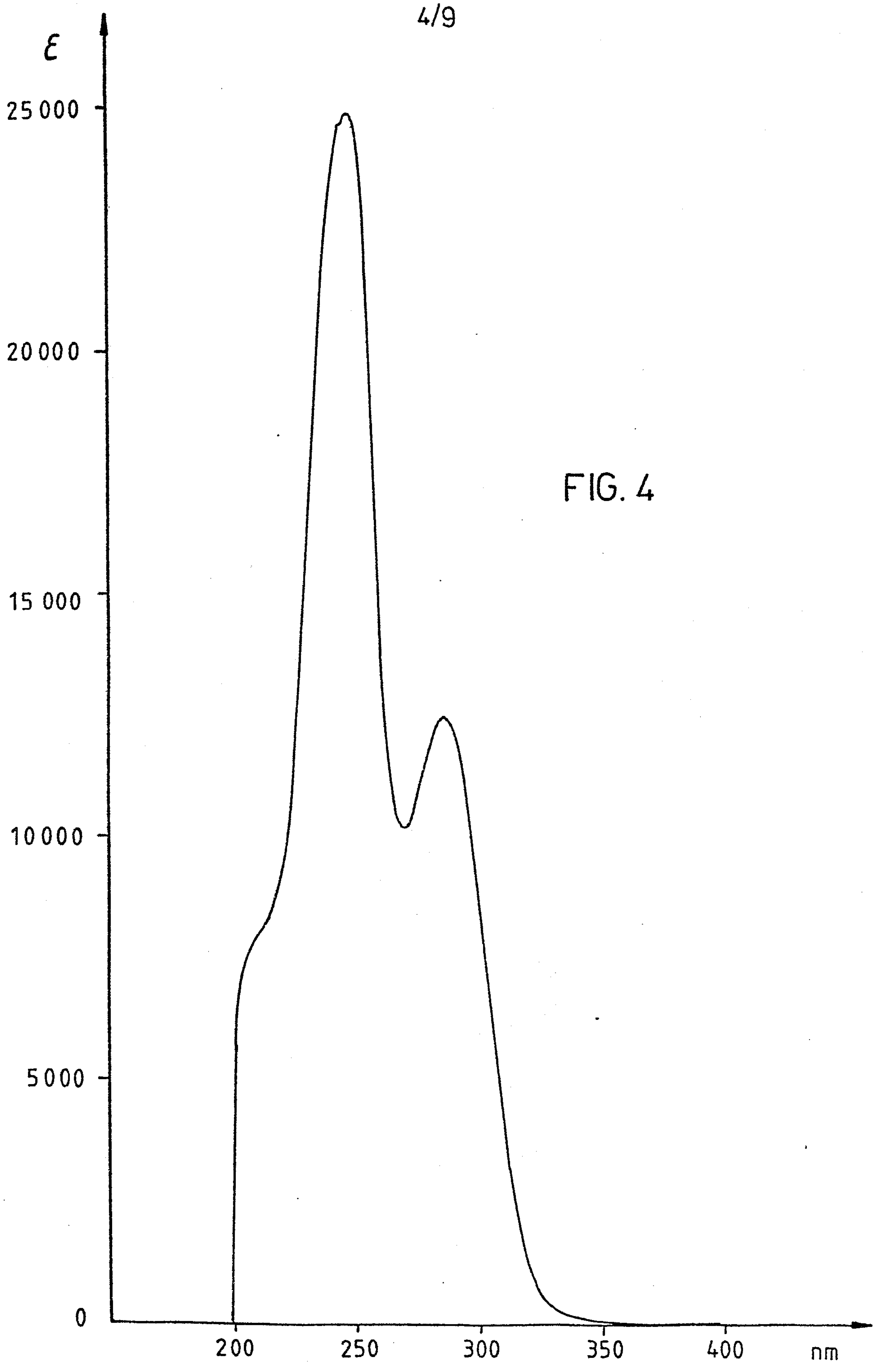


FIG. 4

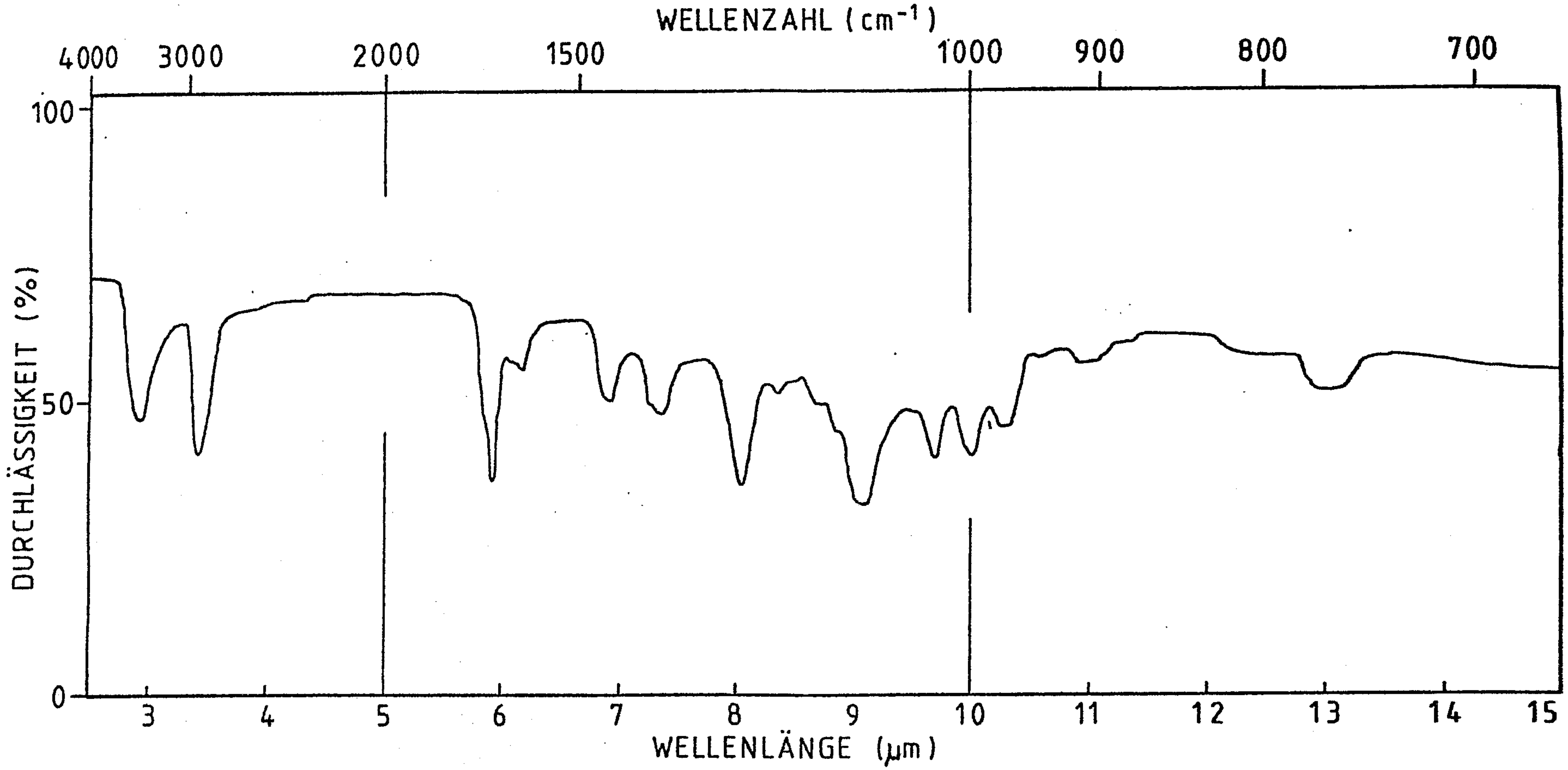
WELLENZAHL (cm⁻¹)
4000 3000 2000 1500 1000 900 800 700
DURCHLÄSSIGKEIT (%)
100 50 0
3 4 5 6 7 8 9 10 11 12 13 14 15
WELLENLÄNGE (μm)

FIG. 5

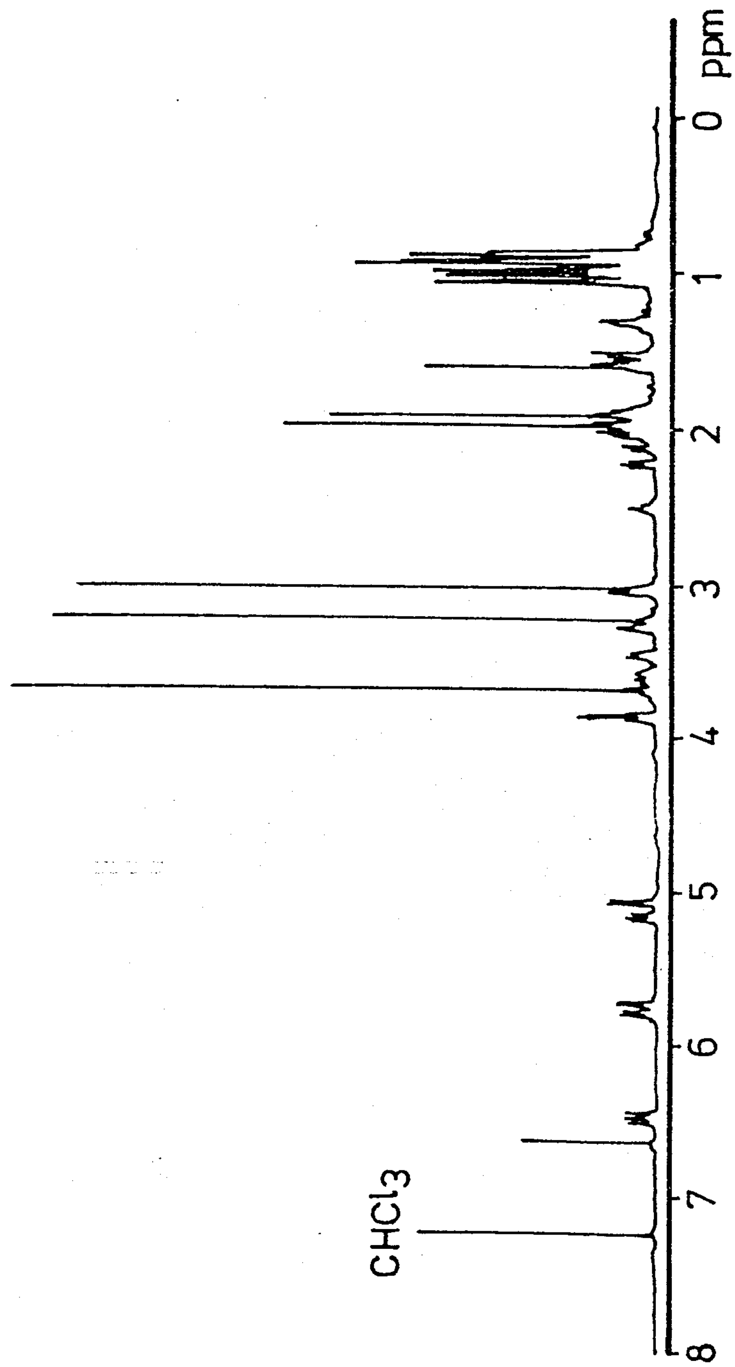


FIG. 6

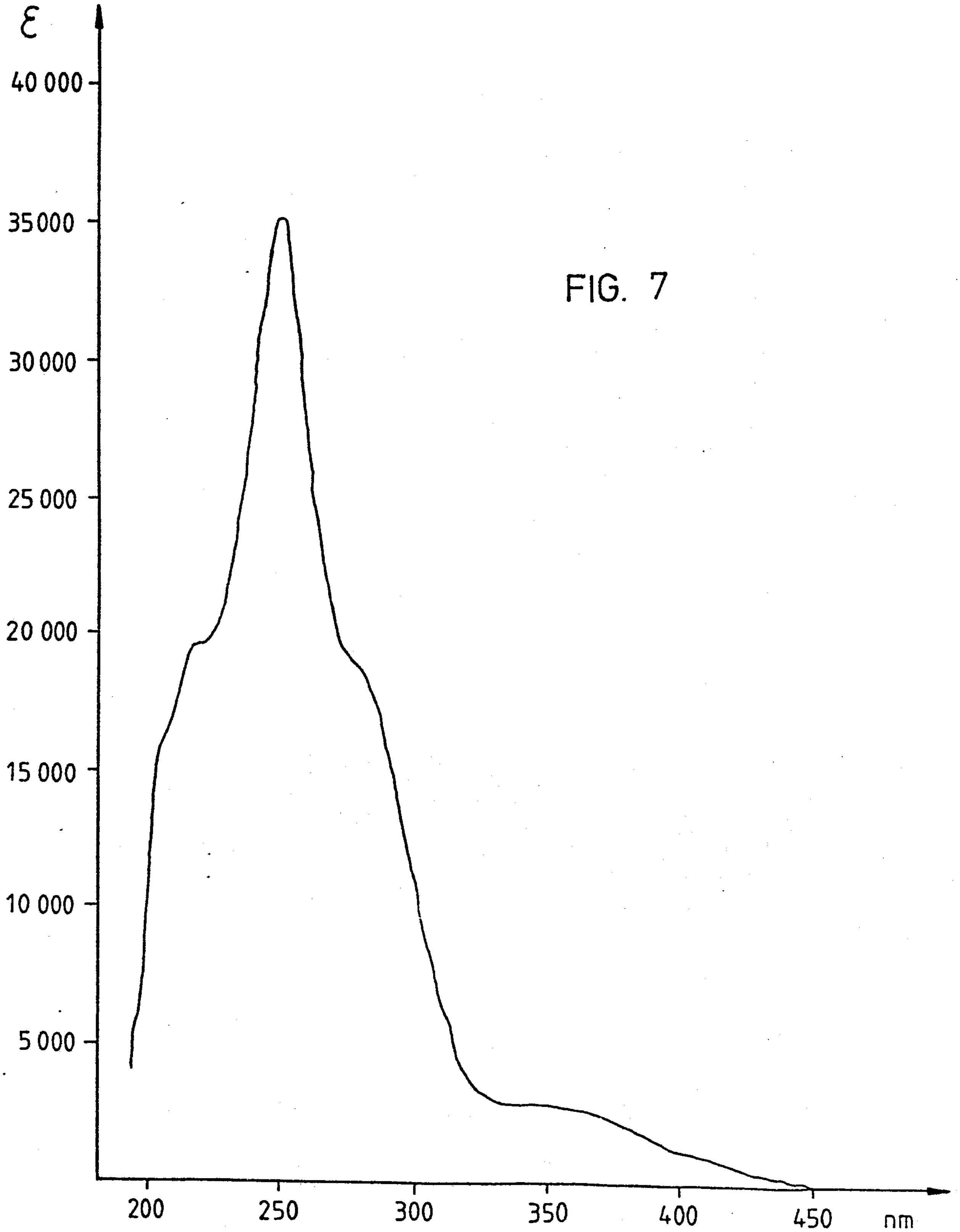
ε
40 000
35 000
30 000
25 000
20 000
15 000
10 000
5 000
200
250
300
350
400
450
nm

FIG. 7

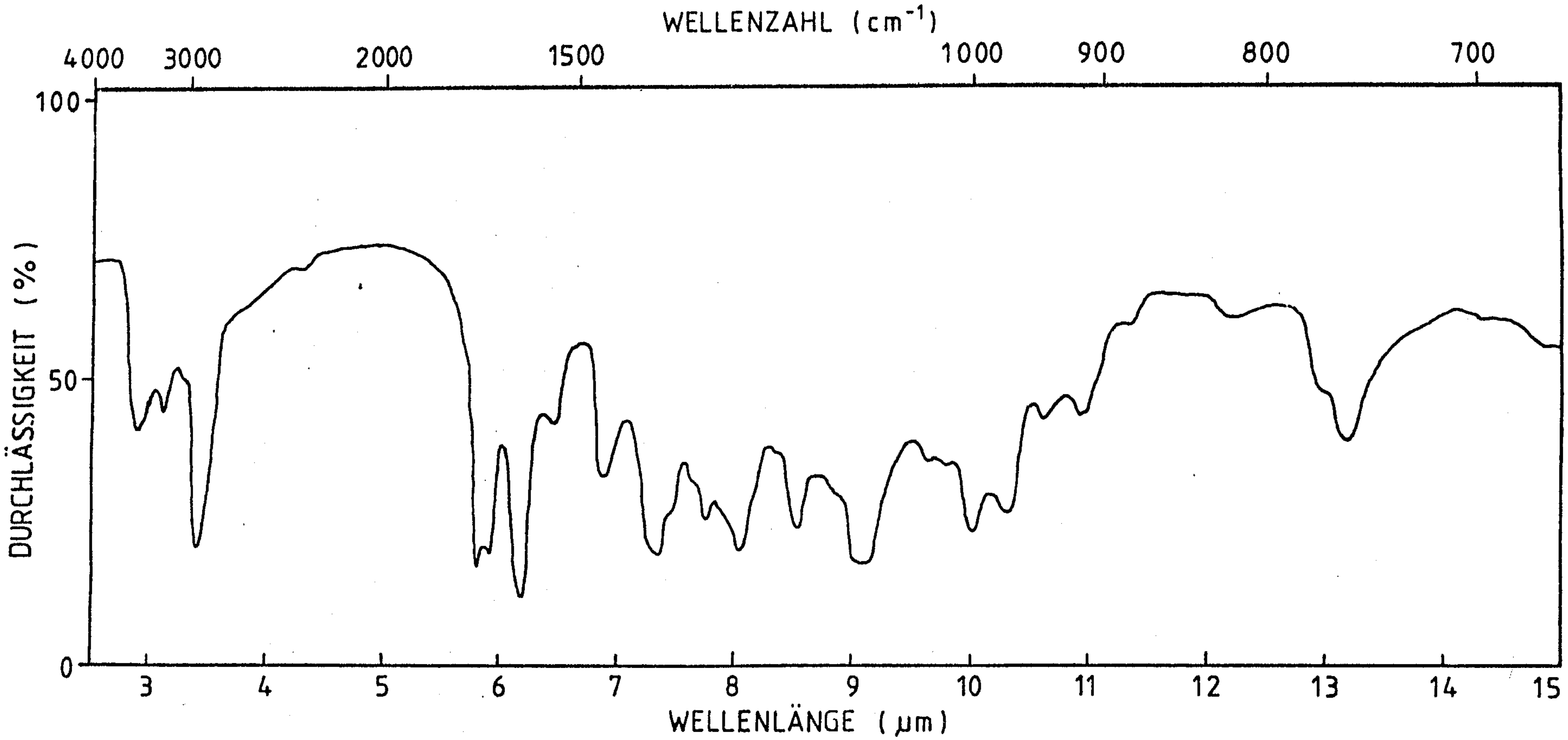
WELLENZAHL (cm⁻¹)
4000
3000
2000
1500
1000
900
800
700
DURCHLÄSSIGKEIT (%)
100
50
0
3
4
5
6
7
8
9
10
11
12
13
14
15
WELLENLÄNGE (µm)

FIG. 8

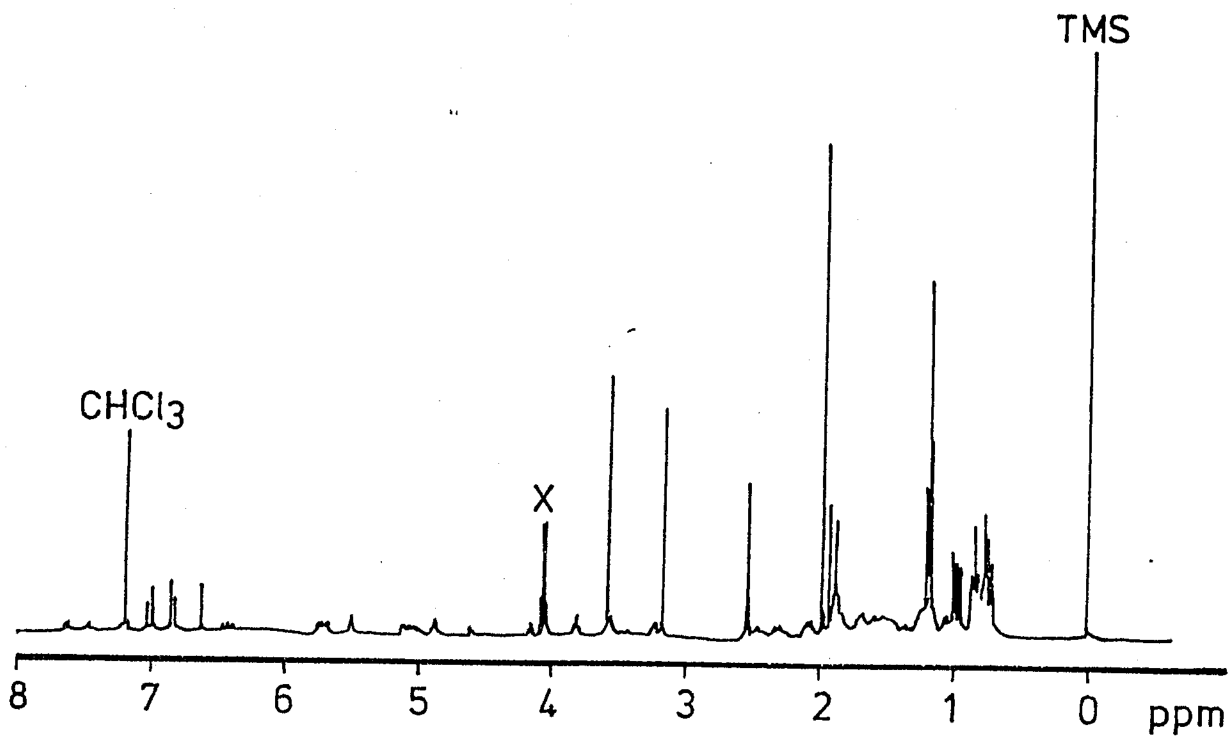


FIG. 9